# EUROPEAN PATENT APPLICATION

(11) **EP 4 458 961 A1**
(43) Date of publication of application: **06.11.2024**
(21) Application number: 21966036.2
(22) Date of filing: 02.12.2021
(51) Int. Cl.: C12N 9/10, C12N 15/54, C12N 15/70, C12N 1/21, C12P 19/18

(54) **ISOLATED POLYPEPTIDE AND USE THEREOF**

(71) Applicant: Glyco-Therapy Biotechnology Co., Ltd., Hangzhou, Zhejiang 310018 (CN)
(72) Inventor: YANG, Yi, Hangzhou, Zhejiang 310018 (CN); CHEN, Ji, Hangzhou, Zhejiang 310018 (CN); JIANG, Xin, Hangzhou, Zhejiang 310018 (CN)
(74) Representative: Weickmann & Weickmann PartmbB
(86) International application number: PCT/CN2021/135057
(87) International publication number: WO 2023/097604

(57) **Abstract**

This present invention relates to an isolated polypeptide and the use thereof for preparing a protein conjugate.

## Description

### TECHNICAL FIELD

This application relates to the field of biomedicine, and in particular to an isolated polypeptide and uses thereof.

### BACKGROUND OF THE INVENTION

Glycosyltransferase, as a type of enzyme that catalyzes the transfer of sugar groups to different receptor molecules (such as proteins, nucleic acids, oligosaccharides, lipids and small molecules), has been widely used in the fields of pharmaceutical products, industrial catalysis, food and agriculture, etc. For example, the synthesis of oligosaccharides through glycosyltransferase has the advantages of high selectivity, mild reaction conditions, and high yield compared to chemical synthesis methods. In addition, modification and transformation of therapeutic proteins (such as antibodies) through glycosyltransferases can effectively change and regulate the efficacy and function of the therapeutic proteins.

In recent years, scientists have gradually expanded the use of glycosyltransferases, such as galactosyltransferase,in the preparation of antibody drug conjugatess (ADCs) by developing and modifying them to to improve their catalytic activity for sugars and their derivatives. ADCs are produced by conjugating small molecule drugs with biological and/or pharmaceutical activity to antibodies. The efficacy of small molecules was targeted-released through antibodies, thereby achieving the function of targeted killing. Efficiently constructing ADCs that are uniform and with good activity remains challenging in this field. Compared with other site-specific conjugation strategies, preparation of ADCs by using glycosyltransferases does not require prior sequence modification of antibodies, which has unique advantages. However, due to the limitations catalytic activity, currently used glycosyltransferases only enables the transfer of very limited reactive functional groups (such as azido and alkynyl) to proteins (e.g., antibodies), which limits their use to a certain extent. On the other hand, due to the limitation of catalytic activity of enzymes, it is hindered to efficiently obtain highly uniform ADCs close to the theoretical MAR (MOI to antibody ratio).

Therefore, it is desired to develop glycosyltransferases with high catalytic activity to transfer more molecules of interest (MOI) to proteins (e.g., antibodies) more efficiently.

### SUMMARY OF THE INVENTION

Fucosylation is an important glycosylation modification in organisms. Fucosyltransferase is a type of enzyme that transfer fucose to a molecule of interest and has many potential application directions.

This application provides a variant (e.g., the isolated polypeptide of this application) based on Helicobacter pylori α-1,3 fucosyltransferase (GenBank accession number AAD07710.1), and the activity of this variant surprisingly has significantly improved in catalyzing the transfer of fucose or fucose derivatives, as compared with the reported α-1,3fucosyltransferase HpFT-4HR (SEQ ID NO: 11) (Wu P. et.al, Proc. Natl. Acad. Sci. USA 2009, 106, 16096). In particular, when catalyzing the transfer of a fucosyl derivative (Fuc*) to a protein (e.g., an antibody) having a glycan chain comprising a structure of -GlcNAc(Fuc)_{b}-GalX, compared to HpFT-4HR ( SEQ ID NO: 11), the variant of this application has greatly improved catalytic activity. This application further provides a method for preparing a protein conjugate (e.g., an ADC) using the variant, and this method can more efficiently and quickly prepare a highly uniform conjugate having a MAR (MOI to antibody ratio) close to the theoretical value. For example, in some cases, the prepared protein conjugate has a MAR of about 3.2-4.0. For example, in some cases, the prepared protein conjugate has a MAR of about 3.6-4.0. For example, in some cases, the prepared protein conjugate has a MAR of about 1.6-2.0. For example, in some cases, the prepared protein conjugate has a MAR of about 1.8-2.0.

In one aspect, this application provides an isolated polypeptide, comprising a catalytically active domain and one or more heptapeptide repeat fragments, the catalytically active domain comprising an amino acid sequence as set forth in SEQ ID NO: 1, the heptapeptide repeat fragment comprising an amino acid sequence as set forth in SEQ ID NO: 2, and in the isolated polypeptide, the number of the heptapeptide repeat fragments being not more than 3. In certain embodiments, in the polypeptide, the catalytically active domain is located at an N-terminus of the heptapeptide repeat fragment.

In certain embodiments, in the polypeptide, the number of the heptapeptide repeat fragments is 1.

In certain embodiments, the polypeptide comprises an amino acid sequence as set forth in SEQ ID NO: 3.

In certain embodiments, in the polypeptide, the number of the heptapeptide repeat fragments is 2, and two the heptapeptide repeat fragments are directly linked to each other.

In certain embodiments, the polypeptide comprises an amino acid sequence as set forth in SEQ ID NO: 4.

In certain embodiments, in the polypeptide, the number of the heptapeptide repeat fragments is 3, and three the heptapeptide repeat fragments are directly linked in sequence.

In certain embodiments, the polypeptide comprises an amino acid sequence as set forth in SEQ ID NO: 5.

In certain embodiments, the polypeptide further comprises a tag sequence. In certain embodiments, the tag sequence is an affinity purification tag.

In certain embodiments, the polypeptide comprises an amino acid sequence as set forth in any one of SEQ ID NOs: 3-5 and 8-10.

In another aspect, this application further provides one or more isolated nucleic acid molecules encoding the isolated polypeptide of this application. For example, the nucleic acid molecule comprises a nucleic acid sequence as set forth in any one of SEQ ID NOs: 25-30.

In another aspect, this application provides a vector comprising the nucleic acid sequence of the nucleic acid molecule of this application.

In another aspect, this application provides a cell comprising and/or expressing the isolated polypeptide of this application, the nucleic acid molecule of this application, and/or the vector of this application.

In another aspect, this application provides a catalytic preparation comprising the isolated polypeptide of this application.

In another aspect, this application provides a composition comprising the isolated polypeptide of this application. In another aspect, this application provides a method for transferring a fucose and/or a fucose derivative, including: using the isolated polypeptide of this application, the nucleic acid molecule of this application, the vector of this application, and/or the cell of this application.

In another aspect, this application provides a use of the isolated polypeptide of this application in the preparation of a protein conjugate.

In another aspect, this application provides a method for preparing a protein conjugate, including: using the isolated polypeptide of this application, the nucleic acid molecule of this application, the vector of this application, the cell of this application, and/or the catalytic preparation of this application.

In certain embodiments, the method includes: in the presence of the isolated polypeptide of this application and/or the catalyst of this application, contacting a donor Q-Fuc* with a protein having a glycan chain comprising a structure of formula (I) to obtain a protein conjugate, the protein conjugate having a glycan chain comprising a structure of formula (II):

-GlcNAc(Fuc)_{b}-GalX, formula (I);

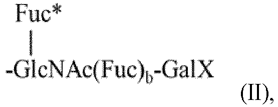

wherein
said GlcNAc is a N-acetylglucosamine;
Fuc is a fucose, and b is 0 or 1;
the GalX is an optionally substituted galactose, that is, the GalX is a galactose or a substituted galactose;
the Fuc* comprises a structure of Fuco-MOI, wherein the structure of Fuco is represented by formula (III): 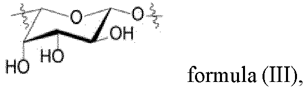 an MOI is a molecule of interest comprising an active substance (AM), the MOI is linked to the left terminus of formula (III), and Q is a ribonucleotide diphosphate.

In certain embodiments, the protein comprises a Fc domain and/or an antigen-binding moiety, the GalX and the GlcNAc are linked via a β-1,4 glycosidic linkage, the Fuc and the GlcNAc are linked via an α-1,6 glycosidic linkage, and the Fuc* and the GlcNAc are linked via an α-1,3 glycosidic linkage.

In certain embodiments, the GalX is a galactose.

In certain embodiments, the GalX is a substituted galactose, and one or more hydroxyls at positions C2, C3, C4 and/or C6 in the galactose are substituted.

In certain embodiments, the GalX is a substituted galactose, and a hydroxyl at position C2 in the galactose is substituted.

In certain embodiments, the GalX is a monosaccharide.

In certain embodiments, the GalX is substituted with a substituent Rg', the Rg¹ is a group selected from the group consisting of: hydrogen, halogen, -NH₂, -SH, -N₃, -COOH, -CN, C₁-C₂₄ alkyl group, C₃-C₂₄ cycloalkyl group, C₂-C₂₄ alkenyl group, C₅-C₂₄ cycloalkenyl group, C₂-C₂₄ alkynyl group, C₇-C₂₄ cycloalkynyl group, C₂-C₂₄ (hetero)aryl group, C₃-C₂₄ alkyl(hetero)aryl group, and C₃-C₂₄ (hetero)arylalkyl group; wherein the alkyl group, cycloalkyl group, alkenyl group, cycloalkenyl group, alkynyl group, cycloalkynyl group, (hetero)aryl group, alkyl(hetero)aryl group and/or (hetero)arylalkyl group are each independently optionally substituted with one or more substituents Rs'(s), and/or each independently optionally spaced by one or more substituents Rs²(s); wherein each of the Rs¹(s) is independently a group selected from the group consisting of: halogen, -OH, -NH₂, -SH, -N₃, -COOH, and -CN; each of the Rs²(s) is independently a group selected from the group consisting of: -O-, -S-, 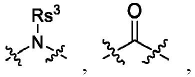 and 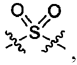 wherein the Rs³ is a group selected from the group consisting of: hydrogen, C₁-C₂₄ alkyl group, C₂-C₂₄ alkenyl group, C₂-C₂₄ alkynyl group, and C₃-C₂₄ cycloalkyl group, wherein the C₁-C₂₄ alkyl group, C₂-C₂₄ alkenyl group, C₂-C₂₄ alkynyl group, and C₃-C₂₄ cycloalkyl group are optionally substituted.

In certain embodiments, the GalX is substituted with a substituent 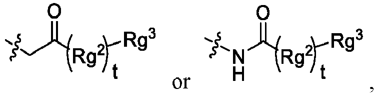 wherein: t is 0 or 1; the Rg² is a group selected from the group consisting of: C₁-C₂₄ alkylene group, C₃-C₂₄ cycloalkylene group, C₂-C₂₄ alkenylene group, C₅-C₂₄ cycloalkenylene group, C₂-C₂₄ alkynylene group, C₇-C₂₄ cycloalkynylene group, C₂-C₂₄ (hetero)arylene group, C₃-C₂₄ alkyl(hetero)arylene group, and C₃-C₂₄ (hetero)arylalkylene group, wherein the alkylene group, cycloalkylene group, alkenylene group, cycloalkenylene group, alkynylene group, cycloalkynylene group, (hetero)arylene group, alkyl(hetero)arylene group and/or (hetero)arylalkylene group are each independently optionally substituted with one or more substituents Rs¹, and/or each independently optionally spaced by one or more substituents Rs²,
said Rg³ is a group selected from the group consisting of: hydrogen, halogen, -OH, -NH₂, -SH, -N₃, -COOH, -CN, C₁-C₂₄ alkyl group, C₃-C₂₄ cycloalkyl group, C₂-C₂₄ alkenyl group, C₅-C₂₄ cycloalkenyl group, C₂-C₂₄ alkynyl group, C₇-C₂₄ cycloalkynyl group, and C₂-C₂₄ (hetero)aryl group; wherein the alkyl group, cycloalkyl group, alkenyl group, cycloalkenyl group, alkynyl group, cycloalkynyl group, and/or (hetero)aryl group are each independently optionally substituted with one or more Rs'(s),
wherein
each of the Rs²(s) is independently selected from the group consisting of: -O-, -S-, 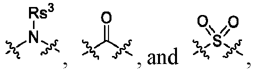 the Rs³ is a group selected from the group consisting of: hydrogen, C₁-C₂₄ alkyl group, C₂-C₂₄ alkenyl group, C₂-C₂₄ alkynyl group, and C₃-C₂₄ cycloalkyl group, wherein the C₁-C₂₄ alkyl group, C₂-C₂₄ alkenyl group, C₂-C₂₄ alkynyl group, and C₃-C₂₄ cycloalkyl group are optionally substituted, and
each of the Rs¹(s) is independently a group selected from the group consisting of: halogen, -OH, -NH₂, -SH, -N₃, - COOH, and -CN.

In certain embodiments, the GlcNAc of the formula (I) is directly linked to an asparagine residue in the protein.

In some embodiments, the glycan chain of the protein comprises a mannose, and the GlcNAc in the formula (I) is directly linked to the mannose in the glycan chain.

In certain embodiments, the protein comprises a Fc domain, and the glycan chain is linked to an asparagine residue of the Fc domain.

In certain embodiments, the protein comprises a Fc domain, and the glycan chain is linked to an asparagine residue of a CH₂ domain in the Fc domain.

In certain embodiments, the protein comprises a Fc domain, and the glycan chain is linked to position N297 of the Fc domain, wherein the position is determined according to EU index numbering in Kabat.

In certain embodiments, the protein is a Fc domain-containing antibody and/or Fc fusion protein.

In certain embodiments, the protein is a monoclonal antibody.

In certain embodiments, the protein is an IgG antibody.

In certain embodiments, the Q is a guanosine diphosphate (GDP), a uridine diphosphate (UDP) and/or a cytidine diphosphate (CDP).

In certain embodiments, the Q is a guanosine diphosphate (GDP).

In certain embodiments, the active substance AM comprises a biologically active substance, a pharmaceutically active substance, a chemically reactive substance and/or an enzymatically reactive substance.

In certain embodiments, the active substance AM is a chemically reactive substance and/or an enzymatically reactive substance.

In certain embodiments, the active substance AM is a functional group X₁, and X₁ comprises a functional group capable of participating in a bioorthogonal ligation reaction.

In certain embodiments, the functional group capable of participating in a bioorthogonal ligation reaction is selected from one or more of the group consisting of: azido, terminal alkynyl, cycloalkynyl, tetrazinyl, 1,2,4-triazinyl, terminal alkenyl, cycloalkenyl, keto, aldehyde, hydroxylamino, mercapto, maleimido and their functional derivatives.

In certain embodiments, the functional group capable of participating in a bioorthogonal ligation reaction is selected from one or more of the group consisting of:

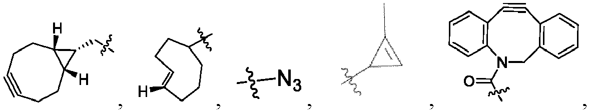

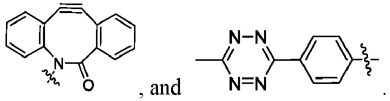

In certain embodiments, the active substance AM is a biologically active substance and/or a pharmaceutically active substance P₁.

In certain embodiments, the P₁ comprises one or more substances selected from the group consisting of: a cytotoxin, an agonist, an antagonist, an antiviral agent, an antibacterial agent, a radioisotope, a radionuclide, a metal chelator, a fluorescent dye, a biotin, an oligonucleotide, and a polypeptide.

In certain embodiments, the active substance AM is a pharmaceutically active substance P₁.

In some embodiments, the P₁ comprises one or more substances selected from the group consisting of: a cytotoxin, an agonist, an antagonist, an antiviral agent, an antibacterial agent, a radioisotope, a radionuclide, a metal chelator, an oligonucleotide, and a polypeptide.

In certain embodiments, the P₁ comprises a cytotoxin.

In certain embodiments, the P₁ comprises a cytotoxin and the cytotoxin is selected from one or more of the group consisting of a DNA damaging agent, a topoisomerase inhibitor, an RNA polymerase inhibitor, and a tubulin inhibitor.

In certain embodiments, the P₁ comprises a cytotoxin, and the cytotoxin is selected from one or more of the group consisting of: pyrrolobenzodiazepine and a derivative thereof, auristatin and a derivative thereof, maytansinoids and a derivative thereof, duocarmycin and a derivative thereof, tubulysin and a derivative thereof, enediyene and a derivative thereof, doxorubicin and a derivative thereof, pyrrole-based kinesin spindle protein inhibitor and a derivative thereof, calicheamicin and a derivative thereof, amanitin and a derivative thereof, and camptothecin and a derivative thereof.

In certain embodiments, the P₁ comprises a cytotoxin and the cytotoxin is selected from one or more of the group consisting of: MMAE and DXd.

In some embodiments, the MOI further comprises a linker L used for linking the AM to the Fuco.

In certain embodiments, the linker L has a structure of J-(Sp)ₙ, wherein n is 0 or 1, J is a connector, Sp is a spacer, and the J is directly linked to the Fuco.

In certain embodiments, the J is a structure selected from the group consisting of: 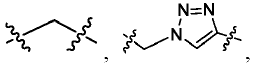 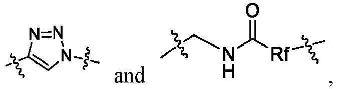 wherein Rf is -CH₂-, -NH- or -O-, wherein the left terminus of the structure is directly linked to the Fuco.

In some embodiments, the J is 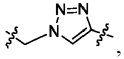 and the left terminus of the structure is directly linked to the Fuco.

In some embodiments, the J is 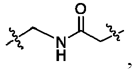 and the left terminus of the structure is directly linked to the Fuco.

In certain embodiments, the spacer Sp comprises a cleavable moiety.

In certain embodiments, the cleavable moiety is selected from one or more of the group consisting of: an acid-labile cleavable moiety, a redox-active cleavable moiety, a photoactive cleavable moiety, and a proteolytically cleavable moiety.

In certain embodiments, the cleavable moiety is selected from one or more of the group consisting of: vc-PAB and GGFG.

In certain embodiments, the spacer Sp does not comprise a cleavable moiety.

In certain embodiments, the Q-Fuc* is a Q-Fuco-L-AM.

In certain embodiments, the Q-Fuc* is a Q-Fuco-J-(Sp)ₙ-AM, wherein n is 0 or 1.

In certain embodiments, the Q-Fuc* is a Q-Fuco-J-(Sp)ₙ-X₁, n is 0 or 1.

In certain embodiments, the Q-Fuc* is a Q-Fuco-J-(Sp)ₙ-P₁, n is 0 or 1.

In certain embodiments, the n is 1. In certain embodiments, the n is 0.

In certain embodiments, the GalX is selected from the group consisting of:

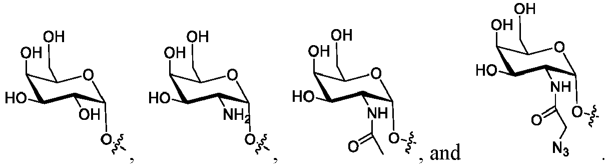

In certain embodiments, the protein conjugate obtained comprises 1 to 20 structures of formula (II).

In certain embodiments, the protein conjugate obtained comprises two or four structures of formula (II).

In certain embodiments, the protein conjugate comprises four structures of formula (II).

In certain embodiments, the structure of the protein conjugate obtained is represented by formula (IV): 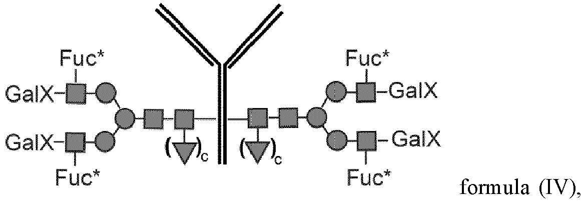 wherein 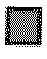 is the N-acetylglucosamine, 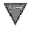 is the fucose, 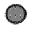 is the mannose, the GalX is the optionally substituted galactose, 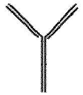 is a Fc domain-containing antibody and/or Fc fusion protein, c is 0 or 1, and the glycan chain is linked to the Fc domain of the Fc domain-containing antibody and/or Fc fusion protein.

In certain embodiments, the method further includes the following step: contacting a Fc domain-containing antibody and/or Fc fusion protein having a G₀(F)_{0,1}, G₁(F)_{0,1} and/or G₂(F)_{0,1} glycoform with a UDP-GalX in the presence of a suitable catalyst to obtain a protein (such as a glycoprotein) having a glycan chain comprising a structure of formula (I), the structure of the protein being represented by formula (V): 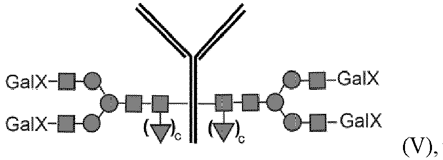 wherein 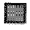 is the N-acetylglucosamine, 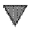 is the fucose, 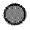 is the mannose, the GalX is the galactose, 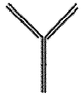 is the Fc domain-containing antibody and/or Fc fusion protein, c is 0 or 1, and the glycan chain is linked to the Fc domain of the Fc domain-containing antibody and/or Fc fusion protein.

In certain embodiments, the method further includes the following step: contacting the Fc domain-containing antibody and/or Fc fusion protein having a G₀(F)_{0,1} glycoform with a UDP-GalX in the presence of a suitable catalyst to obtain a protein (such as a glycoprotein) having a glycan chain comprising a structure of formula (I), the structure of the protein being represented by formula (V): 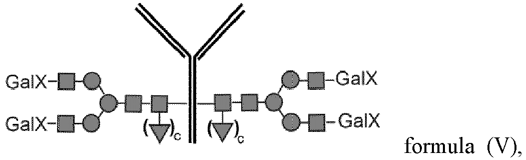 wherein 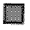 is the N-acetylglucosamine, 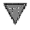 is the fucose, 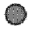 is the mannose, the GalX is the substituted galactose, 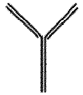 is the Fc domain-containing antibody and/or Fc fusion protein, c is 0 or 1, and the glycan chain is linked to the Fc domain of the Fc domain-containing antibody and/or Fc fusion protein.

In certain embodiments of the method of this application, the protein conjugate may comprise two structures of formula (II).

In certain embodiments, the structure of the protein conjugate is represented by formula (VI): 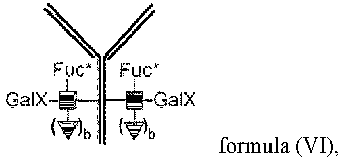 wherein 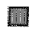 is the N-acetylglucosamine, 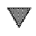 is the fucose, the GalX is the optionally substituted galactose, 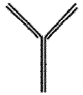 is a Fc domain-containing antibody and/or Fc fusion protein, b is 0 or 1, and the glycan chain is linked to the Fc domain of the Fc domain-containing antibody and/or Fc fusion protein.

In certain embodiments, the method further includes the following steps: treating the Fc domain-containing antibody and/or Fc fusion protein with an endoglycosidase to obtain the Fc domain-containing antibody and/or Fc fusion protein treated; and contacting the treated Fc domain-containing antibody and/or Fc fusion protein with a UDP-GalX in the presence of a suitable catalyst to obtain a glycoprotein having a glycan chain comprising the structure of formula (I), the structure of the glycoprotein being represented by formula (VII): 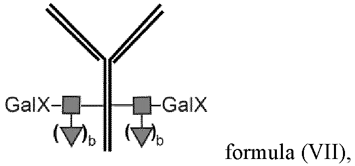 wherein 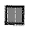 is the N-acetylglucosamine, 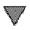 is the fucose, the GalX is the optionally substituted galactose, 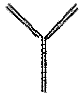 is the Fc domain-containing antibody and/or Fc fusion protein, b is 0 or 1, and the glycan chain is linked to the Fc domain of the Fc domain-containing antibody and/or Fc fusion protein.

In certain embodiments, the method further includes the following steps: treating the Fc domain-containing antibody and/or Fc fusion protein with an endoglycosidase and an α1,6 fucosidase to obtain the Fc domain-containing antibody and/or Fc fusion protein treated; and contacting the treated Fc domain-containing antibody and/or Fc fusion protein with a UDP-GalX in the presence of a suitable catalyst to obtain a glycoprotein having a glycan chain comprising a structure of formula (I), the structure of the glycoprotein being represented by formula (VII): 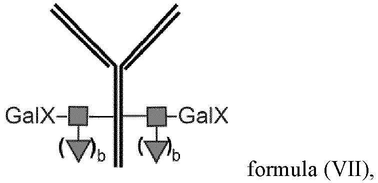 wherein 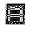 is the N-acetylglucosamine, 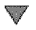 is the fucose, the GalX is the optionally substituted galactose, 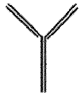 is the Fc domain-containing antibody and/or Fc fusion protein, b is 0, and the glycan chain is linked to the Fc domain of the Fc domain-containing antibody and/or Fc fusion protein. In certain embodiments, the method further includes the following step: reacting the obtained protein conjugate with a Y₁-L'-P₁' to obtain a protein conjugate comprising a biologically active substance and/or a pharmaceutically active substance P₁', wherein the Y₁ is a functional group capable of having a ligation reaction with the X₁, and L' is a linker.

In another aspect, this application further provides a protein conjugate prepared by the method of this application.

In certain embodiments, the protein conjugate of this application has a MAR of about 3.2-4.0 or about 1.6-2.0.

In certain embodiments, the protein conjugate of this application has a MAR of about 3.6-4.0 or about 1.8-2.0.

In another aspect, this application provides a composition comprising one or more protein conjugates of this application.

In certain embodiments, the composition of this application further comprises a pharmaceutically acceptable carrier.

In another aspect, this application provides a method for preventing, alleviating and/or treating a disease or a disorder, the method comprising: administering the protein conjugate of this application and/or the composition of this application to a subject in need.

In another aspect, this application provides a use of the protein conjugate of this application and/or the composition of this application in the preparation of a medicament, the medicament being used for preventing, alleviating and/or treating a disease or a disorder.

Those skilled in the art can easily perceive other aspects and advantages of this application from the following detailed description. Only exemplary embodiments of this application are shown and described in the following detailed description. As those skilled in the art will appreciate, from the content of this application, those skilled in the art can make changes to the disclosed specific embodiments without departing from the spirit and scope of the invention to which this application relates. Accordingly, the descriptions in the drawings and specification of this application are only exemplary and not restrictive.

### BRIEF DESCRIPTION OF THE DRAWING

The specific features of the invention to which this application relates are set forth in the appended claims. The features and advantages of the invention to which this application relates can be better understood with reference to the exemplary embodiments and the accompanying drawings described below in detail. The drawings are described briefly as follows.
Figure 1 illustrates the comparison of the efficiency of different HpFT variants in transferring GDP-Fuc* to Trastuzumab-G₂F.
Figure 2 illustrates the comparison of the efficiency of different HpFT variants in transferring GDP-Fuc* to Trastuzumab-(Fucα1,6)(Galβ1,4)GlcNAc.
Figure 3 illustrates the comparison of the efficiency of different HpFT variants in transferring GDP-Fuc* to Trastuzumab-(Fucα1,6) (GalNAzβ1,4)GlcNAc.
Figure 4 illustrates the comparison of the efficiency of different enzyme variants in transferring GDP-Fuc* to Trastuzumab-(GalNAzβ1,4)GlcNAc.
Figure 5 illustrates the comparison of the efficiency of HpFT-4HR and HFT6 in transferring GDP-Fuc* to Trastuzumab-G₂F and Trastuzumab-(GalNAzβ1,4)GlcNAc. A) The comparison of the efficiency of HpFT-4HR and HFT6 in transferring GDP-Fuc* to Trastuzumab-G₂F. B) The comparison of the efficiency of HpFT-4HR and HFT6 in transferring GDP-Fuc* to Trastuzumab-(GalNAzβ1,4)GlcNAc.
Figure 6 illustrates the comparison of the efficiency of HpFT-2HR and HpFT-4HR in transferring GDP-FAzP₄Biotin and GDP-FAmP₄Biotin to Trastuzumab-G₂F (A) and Trastuzumab-(GalNAzβ1,4)GlcNAc (B).
Figure 7 illustrates the comparison of the efficiency of HpFT-2HR and HpFT-4HR in transferring GDP-FAmSucMMAE to Trastuzumab-G₂F (A) and Durvalumab-G₂F (B).
Figure 8 illustrates the comparison of the efficiency of HpFT-2HR and HpFT-4HR in transferring GDP-FAmSucMMAE to Trastuzumab-(GalNAzβ1,4)GlcNAc (A) and Durvalumab-(GalNAzβ1,4)GlcNAc (B).
Figure 9 illustrates the comparison of the efficiency of HpFT-2HR and HpFT-4HR in transferring GDP-FAmSucMMAE to Trastuzumab-(Galβ1,4)GlcNAc.
Figure 10 illustrates the MS analysis of antibody conjugates prepared using HpFT-2HR.
Figure 11 illustrates some exemplary structures of the Q-Fuc* described in this application.
Figure 12 illustrates structures of some of compounds used in this application.
Figure 13 illustrates the in vitro activity of exemplary protein conjugates of this application.

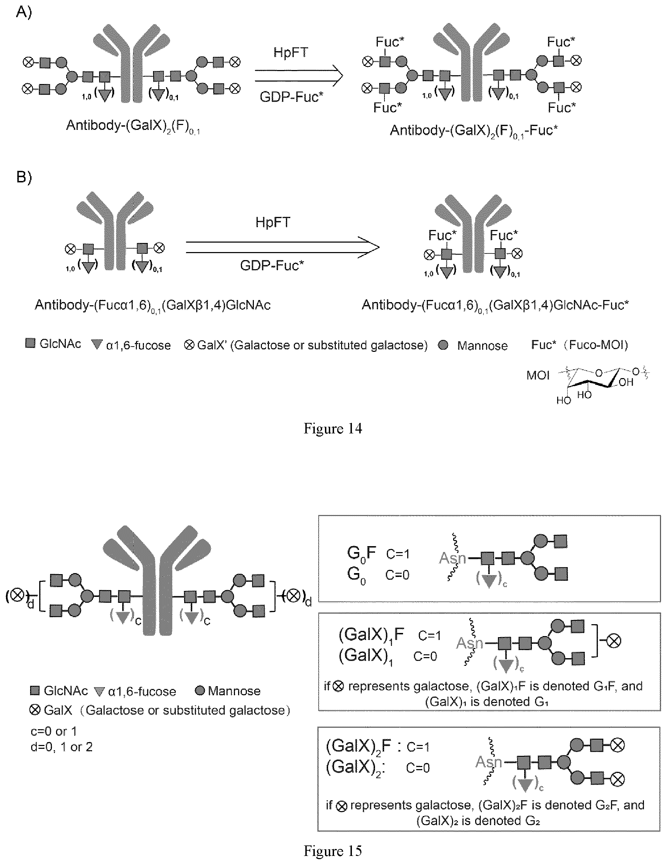

Figure 14 illustrates a schematic diagram of protein conjugates prepared by the method of this application.
Figure 15 illustrates exemplary glycoforms on Fc domain-containing antibodies and/or Fc fusion proteins of this application.

### DETAILED DESCRIPTION OF THE INVENTION

The implementation of the present application will be illustrated in the following specific examples, and other advantages and effects of the present application will be easily known by those familiar with this technology from the content disclosed in the specification.

### Definition of Terms

In this application, the term "tag sequence" generally refers to another molecular entity (e.g., another amino acid sequence tag) that is integrated (e.g., linked) into a protein of interest (e.g., an antibody). For example, the tag sequence may be a detectable label, such as a radiolabeled amino acid or a biotinylated polypeptide, which can be detected by a labeled avidin (e.g., a fluorescently labeled or enzymatically active streptavidin, detectable by optical or colorimetric methods). For example, the tag sequence may include, but is not limited to: radioisotopes or radionuclides (e.g., 3^{H}, 14^{C}, 15^{N}, 35^{S}, 90^{Y}, 99^{Tc}, 111^{In}, 125^{I}, and 131^{I}), fluorescent tags (e.g., FITC, rhodamine, lanthanum phosphor), enzyme tags (e.g. horseradish peroxidase, β-galactosidase, luciferase, and alkaline phosphatase), chemiluminescent labels, biotin groups, preselected polypeptide epitopes recognizable by second receptors (such as leucine zipper complementary sequences, binding sites of secondary antibodies, metal-binding domains, and additional epitopes), magnetic reagents such as gadolinium chelates, toxins such as pertussis toxin, paclitaxel, cytochalasin B, gramicidin D, ethidium bromide, ipecacine, mitomycin, epipodophyllotoxin, thiopodophyllin, vincristine, colchicine, adriamycin, daunorubicin, dihydroxyanthracenedione, mitoxantrone hydrochloride, mitoxantrone, actinomycin D, 1-dehydrotestosterone, glucocorticoids, procaine, tetracaine, lidocaine, propranolol, puromycin and their analogs or homologs. In certain embodiments, the tag sequence may be an affinity purification tag, such as a polyhistidine tag (His tag), Arg tag, FLAG tag, 3xFlag tag, streptavidin tag, nanotag, SBP tag, c-myc tag, S-tag, calmodulin-binding peptide, cellulose-binding domain, chitin-binding domain, GST tag and/or MBP tag.

As used herein, the term "protein conjugate" generally refers to a protein chemically linked to a second moiety (e.g., a therapeutic or cytotoxic agent).

As used herein, the term "isolated polypeptide" generally refers to a polypeptide that 1) has been isolated from at least about 50% of the polynucleotides, lipids, carbohydrates, or other materials with which it is naturally occurring when isolated from the source cell, 2) not linked to the whole or moiety of the molecule to which the "isolated polypeptide" is linked in nature (through covalent or non-covalent interactions), 3) is operably linked (through covalent or non-covalent interactions) to a molecule to which it is not linked in nature, or 4) does not exist in nature. For example, the isolated polypeptide is substantially free of any other contaminating polypeptides or other contaminants that are present in their natural environment and would interfere with their therapeutic, diagnostic, prophylactic or research use.

In this application, the term "directly linked" generally means that the linking site does not comprise additional compounds (e.g., other sugar groups) or connectors. For example, one molecule or entity is directly linked to another one without the presence of another molecule or entity therebetween. For example, the "directly linked" may refer to that a moiety is linked to another moiety without any intermediate moiety or connector. For example, "a GlcNAc is directly linked to an amino acid residue of a protein (e.g., an antibody") generally refers to that the GlcNAc is linked via a covalent bond to an amino acid residue of the protein, for example, via an N-glycosidic linkage and an amide nitrogen linkage to an atom in a side chain of an amino acid (e.g., an asparagine amino acid) of the protein. In this application, when a GlcNAc is "indirectly linked" to an amino acid of the protein, there are usually at least one monosaccharide moiety between the GlcNAc and the amino acid of the protein.

In this application, the term "comprising" generally means including the content following this qualification, but not excluding other content not specifically mentioned, that is, it should generally be understood as an open qualification in this application.

As used herein, the terms "a", "an", "the/said" and similar terms used in the context of the present invention (especially in the context of the claims) should be understood as including both the singular and the plural, unless otherwise indicated herein or the context clearly dictates.

The compounds disclosed in the specification and claims of this application may comprise one or more asymmetric centers, and may exist as different diastereomers and/or enantiomers of said compounds. Unless otherwise stated, the description of any compound in the specification and claims of this application is intended to include all diastereomers and mixtures thereof. Furthermore, unless otherwise stated, description of any compound in this specification and claims is intended to include individual enantiomers as well as any mixtures, racemates or other forms of the enantiomers. When the structure of a compound is described as a specific enantiomer, it should be understood that the invention of this application is not limited to the specific enantiomer.

The compound may exist in different tautomeric forms. Unless otherwise stated, the compounds of the present invention are intended to include all tautomeric forms. When the structure of a compound is described as a specific tautomer, it should be understood that the invention of this application is not limited to the specific tautomer.

An unsubstituted alkyl has a general formula of CₙH₂ₙ₊₁ and may be linear or branched. An unsubstituted alkyl may further comprise a cyclic moiety and thus have the corresponding general formula of CₙH₂ₙ₋₁. Optionally, said alkyl is substituted with one or more substituents as further specified in this application. Examples of the alkyl include, for example, methyl, ethyl, propyl, 2-propyl, tert-butyl, 1-hexyl, and 1-dodecyl.

An aryl may comprise six to twelve carbon atoms and may comprise monocyclic and bicyclic structures. Optionally, the aryl may be substituted with one or more substituents as further specified herein. Examples of aryl are phenyl and naphthyl.

An arylalkyl and an alkylaryl may comprise at least seven carbon atoms and may comprise monocyclic and bicyclic structures. Optionally, said arylalkyl and alkylaryl may be substituted with one or more substituents as further specified herein. The arylalkyl may be, for example, benzyl. The alkylaryl may be, for example, 4-tert-butylphenyl. In this application, the term "heteroaryl" generally refers to an aromatic monocyclic or polycyclic group comprising five to twelve atoms, which has at least one aromatic ring comprising one, two or three cyclic heteroatoms selected from N, O, and S, the remaining cyclic atoms being C atoms. One or two cyclic carbon atoms of the heteroaryl may be substituted with a carbonyl(s).

A heteroarylalkyl group and an alkylheteroaryl group comprise at least three carbon atoms (i.e., at least C3) and may comprise monocyclic and bicyclic structures. Optionally, the heteroaryl may be substituted with one or more substituents further specified herein.

When the aryl is denoted as a (hetero)aryl group, this notation is intended to include both aryl and heteroaryl. Similarly, the alkyl(hetero)aryl is intended to include alkylaryl and alkylheteroaryl, and the (hetero)arylalkyl is intended to include arylalkyl and heteroarylalkyl. Therefore, the C₂-C₂₄ (hetero)aryl should be understood as including both C₂-C₂₄ heteroaryl and C₆-C₂₄ aryl groups. Similarly, the C₃-C₂₄ alkyl(hetero)aryl is intended to include C₇-C₂₄ alkylaryl and C₃-C₂₄ alkylheteroaryl groups, and the C₃-C₂₄ (hetero)arylalkyl is intended to include C₇-C₂₄ arylalkyl and C₃-C₂₄ heteroarylalkyl groups.

Unless otherwise stated, the alkyl, alkenyl, alkene, alkyne, (hetero)aryl, (hetero)arylalkyl and alkyl(hetero)aryl may be substituted with one or more substituents selected from the group consisting of: C₁-C₁₂ alkyl, C₂-C₁₂ alkenyl, C₂-C₁₂ alkynyl, C₃-C₁₂ cycloalkyl, C₅-C₁₂ cycloalkenyl, C₇-C₁₂ cycloalkynyl, C₁-C₁₂ alkoxy, C₂-C₁₂ alkenyloxy, C₂-C₁₂ alkynyloxy, C₃-C₁₂ cycloalkoxy, halogen, amino, oxo, wherein the alkyl, alkenyl, alkynyl, cycloalkyl, alkoxy, alkenyloxy, alkynyloxy and cycloalkoxy are optionally substituted, and the alkyl, alkoxy, cycloalkyl and cycloalkoxy are optionally spaced or interrupted by one or more heteroatoms selected from O, N and S.

As used herein, the term "alkynyl" generally comprises carbon-carbon triple bonds. The unsubstituted alkynyl comprising one triple bond has a general formula of CnH₂ₙ₋₃. The terminal alkynyl is one in which the triple bond is at the terminal of the carbon chain. Optionally, the alkynyl is substituted with one or more substituents as further specified herein, and/or spaced or interrupted by heteroatoms selected from O, N and S. Examples of the alkynyl include, for example, ethynyl, propynyl, butynyl, and octynyl.

As used herein, the term "cycloalkynyl" generally refers to an unsaturated monocyclic, bicyclic or tricyclic hydrocarbon ring having a specified number of carbon atoms and one or more carbon-carbon triple bonds. For example, C₇-C₁₂ cycloalkynyl refers to a cycloalkynyl having seven to twelve carbon atoms. In certain embodiments, the cycloalkynyl has one carbon-carbon triple bond in its ring. In other embodiments, the cycloalkynyl has more than one carbon-carbon triple bond in its ring. Representative examples of the cycloalkynyl include, but are not limited to, cycloheptynyl and cyclooctynyl.

As used herein, the term "heterocycloalkynyl" generally refers to a cycloalkynyl spaced or interrupted by heteroatoms selected from O, N and S. Optionally, the heterocycloalkynyl is substituted with one or more substituents further specified herein. An example of the heterocycloalkynyl is azacyclooctynyl.

When the alkyl, (hetero)aryl, alkyl(hetero)aryl, (hetero)arylalkyl, or (hetero)cycloalkynyl group is optionally substituted, said group is independently optionally substituted with one or more substituents independently selected from the group consisting of: C₁-C₁₂ alkyl, C₂-C₁₂ alkenyl, C₂-C₁₂ alkynyl, C₃-C₁₂ cycloalkyl, C₁-C₁₂ alkoxy, C₂-C₁₂ alkenyloxy, C₂-C₁₂ alkynyloxy, C₃-C₁₂ cycloalkoxy, halogen, amino, oxo and silyl, wherein the alkyl, alkenyl, alkynyl, cycloalkyl, alkoxy, alkenyloxy, alkynyloxy and cycloalkoxy are optionally substituted, and the alkyl, alkoxy, cycloalkyl and cycloalkoxy are optionally spaced or interrupted by one or more heteroatoms selected from O, N and S.

In this application, the term "sugar" generally refers to monosaccharides such as glucose (Glc), galactose (Gal), mannose (Man) and fucose (Fuc). The term "sugar derivative" used herein generally refers to derivatives of monosaccharides, i.e., monosaccharides comprising substituents and/or functional groups. Examples of the sugar derivative include amino sugars and sugar acids such as glucosamine (GlcNH₂), galactosamine (GalNH₂), N-acetylglucosamine (GlcNAc), N-acetylgalactosamine (GalNAc), sialic acid (Sia) (also known as N-acetylneuraminic acid (NeuNAc)), and N-acetylmuramic acid (MurNAc), glucuronic acid (GlcA), and iduronic acid (IdoA). Examples of the sugar derivative also include compounds represented herein as GalX, which may be a galactose or a galactose derivative. Examples of the sugar derivative also include compounds represented herein as Fuc*, which may be a fucose derivative.

As used herein, the term "nucleotide" is used in its ordinary scientific sense. The term "nucleotide" refers to a molecule composed of a nucleobase, a five-carbon sugar (ribose or 2-deoxyribose) and one, two, or three phosphate groups. Without a phosphate group, the nucleobase and sugar make up the nucleoside. Therefore, the nucleotide is also called nucleoside monophosphate, nucleoside diphosphate, or nucleoside triphosphate. The nucleobase may be adenine, guanine, cytosine, uracil, or thymine. Examples of the nucleotide include ribonucleotide diphosphates such as uridine diphosphate (UDP), guanosine diphosphate (GDP), thymidine diphosphate (TDP), cytidine diphosphate (CDP), and cytidine monophosphate (CMP).

As used herein, the term "protein" and "protein" is generally used in its common scientific sense. As used herein, a polypeptide comprising about 10 or more amino acids is considered as a protein. The protein may include natural amino acids, but may also include unnatural amino acids.

As used herein, the term "glycoprotein" is used in its common scientific sense and refers to a protein comprising one or more monosaccharide or oligosaccharide chains ("glycan chains") covalently bonded to the protein. The glycan chain may be linked to a hydroxyl group of the protein (O-linked-sugar chain), for example to a hydroxyl group of serine, threonine, tyrosine, hydroxylysine or hydroxyproline; or to an amide group of a protein (N-glycoprotein), such as asparagine or arginine; or to a carbon in a protein (C-glycoproteins), such as tryptophan. The glycoprotein may comprise more than one glycan chain, may comprise a combination of one or more monosaccharide chains and one or more oligosaccharide chains, and may comprise a combination of N-linked, O-linked and C-linked glycan chains. It is estimated that over 50% of all proteins have some forms of glycosylation and thus can be described as glycoprotein

The term "glycan chain", as used herein, generally refers to a monosaccharide or oligosaccharide chain linked to a protein. Therefore, the term "glycan chain" refers to the carbohydrate moiety of a glycoprotein. The glycan chain is linked to the protein through the C-1 carbon of one sugar and said glycan chain may be free of other substituents (monosaccharides) or may be further substituted on one or more of their hydroxyl groups (oligosaccharides). A naturally occurring glycan typically comprises one to about ten sugar moieties. However, when a longer glycan is linked to a protein, the glycan is also considered as the glycan chain described herein.

The glycan chain of the glycoprotein may be a monosaccharide. Typically, the monosaccharide chain of the glycoprotein consists of a single N-acetylglucosamine (GlcNAc), glucose (Glc), mannose (Man), or fucose (Fuc) covalently bonded to the protein. The glycan chain may also be an oligosaccharide.

The oligosaccharide chain of the glycoprotein may be linear or branched. In the oligosaccharide, the sugar directly linked to the protein is called core sugar. In the oligosaccharide, the sugar not directly linked to the protein and linked to at least two other sugars is called endosaccharide. In the oligosaccharide, the sugar not directly linked to the protein but linked to a single other sugar, that is, not linked to another sugar on one or more of its other hydroxyl groups, is called terminal sugar. For the avoidance of doubt, multiple terminal sugars may be present in the oligosaccharide of the glycoprotein, but typically there is only one core sugar.

The glycan chain may be an O-linked glycan chain, an N-linked glycan chain, or a C-linked glycan chain. In the O-linked glycan chain, a monosaccharide or oligosaccharide chain is bonded to an O atom in an amino acid of said protein, usually via the hydroxyl of serine (Ser) or threonine (Thr). In the N-linked glycan chain, a monosaccharide or oligosaccharide chain is bonded to the protein via a N atom in an amino acid of said protein, typically via an amide nitrogen on the side chain of asparagine (Asn) or arginine (Arg). In the C-linked glycan chain, a monosaccharide or oligosaccharide glycan chain is bonded to a C atom in an amino acid of said protein, usually to the C atom of tryptophan (Trp).

The terminal of an oligosaccharide directly linked to a protein is called the reducing terminal of the glycan chain. The other end of said oligosaccharide is called the non-reducing terminal of the glycan chain.

For the O-linked glycan chains, there are many different chains. Naturally occurring O-linked glycan chains typically have a serine or threonine-linked α-O-GalNAc moiety, which may also be substituted with a galactose, a sialic acid and/or a fucose. A hydroxylated amino acid carrying glycan chain substituents can be a moiety of any amino acid sequence in a protein.

For the N-linked glycan chains, there are many different chains. Naturally occurring N-linked glycan chains typically have an asparagine-linked β-N-GlcNAc moiety, which in turn is further linked to a β-GlcNAc on its C4-OH and then further to a β-Man on its C4-OH, and then further to an α-Man on its C3-OH and C6-OH, thus form a pentasaccharide Man₃GlcNAc₂. The core GlcNAc moiety may also be linked to an α-Fuc on its C6-OH. The pentasaccharide Man₃GlcNAc₂ is a common oligosaccharide framework for most N-linked glycoproteins and can be further linked to other sugars, including but not limited to Man, GlcNAc, Gal, and sialic acid. The asparagine modified with a glycan chain on the side chain is usually a moiety of the sequence Asn-X-Ser/Thr, where X is any amino acid except proline and the Ser/Thr is serine or threonine.

In this application, the term "antibody" generally refers to a protein or an antigen-binding fragment thereof that is produced by the immune system and capable of recognizing and binding to a specific antigen. The antibody is an example of glycoproteins. The term "antibody" is used in this application in its broadest sense and specifically includes monoclonal antibodies, polyclonal antibodies, dimers, multimers, multispecific antibodies (e.g., bispecific antibodies), antibody fragments, and double-chain, and single-chain antibodies. The antibody also includes human antibodies, humanized antibodies, chimeric antibodies, and antibodies that specifically bind to cancer antigens. In this application, the term "antibody" generally includes intact antibodies, but also includes antibody fragments, such as antibody Fab fragments, (Fab')₂, Fv fragments or Fc fragments from cleaved antibodies, scFv-Fc fragments, minibodies, nanobodies, diabodies, or scFv. In addition, the term "antibody" also includes engineered or genetically modified antibodies and/or derivatives of the antibodies. In some embodiments, the antibody is referred to as an immunoglobulin and includes various classes and isotypes, such as IgA (IgAl and IgA2), IgD, IgE, IgM, and IgG (IgGl, IgG3 and IgG4), etc. The term "antibody" as used herein refers to polyclonal and monoclonal antibodies, as well as functional fragments thereof. The antibody includes modified or derivatized antibody variants that retain the ability to specifically bind to an epitope. The antibody is capable of selectively binding to a target antigen or epitope. In this application, the antibody may be from any origin, such as mouse or human, including a chimeric antibody thereof. For example, the antibody may be humanized.

The term "humanized antibody", as used herein, generally refers to an antibody that comprises some or all of the CDRs from a non-human animal antibody, and the framework and constant regions of the antibody comprise amino acid residues from human antibody sequences.

Antibodies, antibody fragments and genetically modified antibodies can be obtained by methods known in the art. Suitable commercially available antibodies include, but are not limited to, Rituximab, basiliximab, palivizumab, infliximab, trastuzumab, alemtuzumab, adalimumab, tositumomab-I131, cetuximab, ibrituximabtiuxetan, omasozumab, bevacizumab, natalizumab, pantumumab, eculizumab, golimumab, canakinumab, catumaxomab, ustekinumab, tocilizumab, ofatumumab, denosumab, belimumab, ipilimumab, Durvalumab, and brentuximab.

As used herein, the term "treatment" generally refers to achieving a desired pharmacological and/or physiological effect. The effect may be prophylactic in terms of complete or partial prevention of a disease or its symptoms, and/or may be therapeutic in terms of partial or complete cure of the disease and/or side effects caused by the disease. "Treatment", as used herein, encompasses any treatment of a disease in mammals, particularly humans, and includes preventing the disease from occurring in a subject who may be susceptible to the disease but has not yet been diagnosed as having the disease; inhibiting the disease, i.e. preventing its progression; and relieving the disease, i.e. causing its regression.

The term "conjugate" as used herein generally refers to any substance formed from separate moieties joined together. In the conjugate, the separate moieties may be linked to each other at one or more active sites. Moreover, the separate moieties may be associated or linked to each other, covalently or non-covalently, and exhibit various stoichiometric molar ratios. The conjugate of this application may include peptides, polypeptides, proteins, prodrugs that are metabolized to active agents in vivo, polymers, nucleic acid molecules, small molecules, binding agents, mimetic agents, synthetic drugs, inorganic molecules, organic molecules, and/or or radioisotopes.

The term "Fc fragment", as used herein, generally refers to the C-terminus region of an immunoglobulin heavy chain, which may be produced by papain digestion of an intact antibody. The Fc domain may be a native sequence Fc domain or a variant Fc domain. The Fc domain of an immunoglobulin generally comprises two constant domains, i.e., a CH2 domain and a CH3 domain, and optionally comprises a CH4 domain and/or a hinge region. As used herein, the term "Fc domain" includes any polypeptide (or nucleic acid encoding such a polypeptide), regardless of the manner in which it is produced.

The term "Fc fusion protein", as used herein, generally refers to a fusion protein comprising a Fc domain and other moieties (e.g., other polypeptides or proteins). In certain embodiments, the other moieties may be biologically active proteins. For example, the other moieties may be therapeutic proteins. For example, the other moieties may be cytokines.

As used herein, the term "GlcNAc", or "N-acetylglucosamine", can be used interchangeably, and generally refers to an amide derivative of the monosaccharide glucose, that is usually linearly polymerized through β-(1,4) linkages. Glycosylation generally refers to the reaction in which a carbohydrate, i.e., a glycosyl donor, is attached to a hydroxyl or other functional group of another molecule (a glycosyl acceptor). In some embodiments, glycosylation mainly refers in particular to the enzymatic process that attaches glycans to proteins, or other organic molecules. The glycosylation in protein can be modified in terms of glycosylation linkage, glycosylation structure, glycosylation composition and/or glycosylation length, etc. Glycosylation may comprise N-linked glycosylation, O-linked glycosylation, phosphoserine glycosylation, C-mannosylation, formation of GPI anchors (glypiation), and/or chemical glycosylation. Correspondingly, a glycosylated oligosaccharide of a protein may be a N-linked oligosaccharide, O-linked oligosaccharide, phosphoserine oligosaccharide, C-mannosylated oligosaccharide, glycosylated oligosaccharide, and/or chemical oligosaccharide.

The term "monoclonal antibody", as used herein, generally refers to an antibody obtained from a population of substantially homogeneous antibodies, i.e., the individual antibodies comprising the population are identical except for possible naturally occurring mutations and/or post-translational modifications (e.g., isomerizations, amidations) that may be present in minor amounts.

The term "IgG", as used herein, generally refers to an immunoglobulin or a functional derivative thereof. Those skilled in the art will appreciate that immunoglobulin heavy chains are classified as gamma, mu, alpha, delta, or epsilon, (γ, µ, α, δ, ε) with some subclasses among them (e.g., γ1-γ4 or α1-α2). It is the nature of this chain that determines the "isotype" of the antibody as IgG, IgM, IgD, IgA, or IgE, respectively. The immunoglobulin subclasses (subtypes), e.g., IgG1, IgG2, IgG3, IgG4, IgA1, IgA2, etc. are well characterized and are known to confer functional specialization. Human IgG is typically characterized by glycosylation at position Asn297 (numbered according to Kabat's EU) in the heavy chain CH2 region of the Fc domain.

In this application, the term "Asn297", or "N297", can be used interchangeably, is the Asparagine at site 297 (numbered according to Kabat's EU numbering convention) of an antibody Fc fragment. Asn297 may be attached with one or more oligosaccharides.

The term "Fuc*α1,3GlcNAc linkage", as used herein, generally refers to a linkage between Fuc of the Fuc* and a GlcNAc, linking the C1 position of Fuco to the C3 position of GlcNAc, for example, typically via an -O- linkage. The term "Galβ1,4GlcNAc linkage", as used herein, generally refers to a linkage between an optionally substituted galactose GalX and a GlcNAc, linking the C1 position of GalX to the C4 position of GlcNAc, for example, typically via an -O- linkage.

The terms "molecule of interest" and "MOI", as used herein, are used interchangeably and generally refer to a molecule with a desired characteristic. The desired characteristic may be a physical characteristic or a chemical characteristic, for example, reactive activity, stability, solubility, binding activity, inhibiting activity, toxicity and/or degradability, etc. The MOI may comprise any substance with a desired biologically active and/or reactive functional group that may be used to a conjugate drug into the protein conjugate of this application. For example, a MOI may comprise an active substance (AM). For example, the active substance may be a therapeutical agent, a diagnosis agent, a pharmacological agent and/or a bioactive agent, e.g., a cytotoxin, a cytostatic agent, a radioisotope or radionuclide, a metal chelator, an oligonucleotide, an antibiotic, a fluorophore, a biotin tag, a peptide, a protein, or any combination thereof. In some cases, the active substance could be a chemically active substance. For example, the chemically active substance may be a chemically functional moiety that could react with another chemically functional moiety to form a covalent bond. For example, a chemically active substance may be able to participate in a ligation reaction. In some cases, the active substance could be an enzymatically active substance that could react with the corresponding functional group in the presence of an enzyme to form a covalent bond.

The term "MAR", as used herein, also refers to "MOI to antibody ratio", which is usually used for representing the ratio of the number of MOIs to the number of protein molecules in a protein conjugate (such as an antibody conjugate). On the other hand, in the case where the MOIs comprise drug molecules (Drug), the MAR is also called DAR ("Drug to antibody ratio"), which is usually used for representing the ratio of the number of drug molecules to the number of protein molecules in a protein conjugate (such as an antibody conjugate).

The term "functional group", as used herein, generally refers to a group capable of reacting with another group. The functional group can be used to link an agent (e.g., an agent without a reactive activity or with a low reactive activity) into the protein conjugate of this application. For example, the functional group may be a chemical group or a residue having chemical and/or enzymatic reactivity. In some embodiments, the functional group may be a group capable of reacting in a ligation reaction.

The term "fucosyltransferase", as used herein, generally refers to an enzyme or a functional fragment or variant thereof that can transfer a L-fucose sugar from a fucose donor substrate (such as guanosine diphosphate-fucose) to an acceptor substrate. The acceptor substrate may be another glycan such as a glycan comprising a GlcNAc-Gal(LacNAc), as in the case of N-glycosylation, or in the case of O-glycosylation. The term "fucosyltransferase" may comprise any functional fragments thereof, or a catalytic domain thereof, as well as functional variants (such as, mutants and isoforms) having a catalytically active domain. The example of fucosyltransferase may be an α-1,3 fucosyltransferase, which recognizes GlcNAc-Gal as a substrate. The term "fucosyltransferase" may be derived from various species, such as mammals (e.g., humans), bacteria, nematodes or trematodes.

The term "pharmaceutically active substance", as used herein, generally refers to any substance that is pharmaceutically useful or pharmaceutically effective. In this application, the pharmaceutically active substance may not comprise a detecting agent (e.g., an agent with a detectable physical or a chemical moiety, and/or only be used for detective purpose in this application). In this application, the pharmaceutically active substance may not comprise a fluorescent label. For example, the pharmaceutically active substance may be an agent capable of alleviating, treating, preventing a disease, or delaying a disease process. The disease may be a disease associated with abnormal cell proliferation and/or cellular dysfunction. For example, the disease may be a tumor and/ or a disease of immune system.

The term "bioorthogonal ligation reaction", as used herein, generally refers to the chemical reaction for preparing the protein conjugate of this application. The reaction occurs specifically between a first functional group located at a specific position in the protein (e.g., on an oligosaccharide of the protein) and a second functional group corresponding to part of said functional group. The first functional group and the second functional group are generally referred to as a bioorthogonal ligation reaction pair. Typically, the first functional group located at a specific position in a protein is easily distinguished from other groups in other parts of the protein. Typically, the second functional group will not react with other parts of the protein except for the first functional group at a specific location. For example, an azido is a functional group capable of participating in a bioorthogonal ligation reaction. The DBCO or BCN group complementary thereto can react specifically with the azido without cross-reacting with other groups in the protein. Many chemically reactive functional groups with suitable reactivity, chemoselectivity, and/or biocompatibility can be used in bioorthogonal ligation reactions. The group capable of participating in a bioorthogonal ligation reaction may be selected from, but are not limited to, azido, terminal alkynyl, cycloalkynyl, tetrazinyl, 1,2,4-triazinyl, terminal alkenyl, cycloalkenyl, keto, aldehyde, hydroxylamino, mercapto, N-maleimido and their functional derivatives (see Bertozzi CR, et.alAngew. Chem. Int. Ed., 2009, 48 , 6974; Chin JW, et. alACS Chem. Biol. 2014, 9, 16; van Del FL, et.al Nat. Commun., 2014, 5, 5378; Prescher JA, et.al Acc. Chem. Res. 2018, 51,1073; Devaraj NK. ACS Cent. Sci. 2018,4,952; Liskamp RMJ, et.al Chem. Sci., 2020,11,9011). The functional derivatives here may refer to modified functional groups, which have similar or higher reactivity than unmodified functional groups. For example, the methylene azido 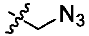 can be a functional derivative of azido

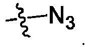

As used herein, the "functional variant" of a parent polypeptide or protein generally has substantial or significant sequence identity or similarity with the parent polypeptide or protein, and the functional variant retains at least part of functions of the parent polypeptide or protein. For example, the functional variant of an enzyme retains enzymatic activity to a similar extent, to the same extent, or to a greater extent than a parent enzyme. With respect to a parent polypeptide or protein, the functional variant may be, for example, about 80% or more, about 90% or more, about 95% or more, about 96% or more, about 97% or more, about 98% or more, or about 99% or more sequence identity to the parent polypeptide or protein in terms of amino acid sequence. In some cases, the functional variant may be a polypeptide that differs from the parent polypeptide or protein by at least one amino acid. For example, the functional variant may be obtained by adding, deleting, or substituting one or more amino acids, such as 1-200, 1-100, 1-50, 1-40, 1-30, 1-20, 1-15, 1-14, 1-13, 1-12, 1-11, 1-10, 1-9, 1-8, 1-7, 1-6, 1- 5, 1-4, 1-3 or 1-2 amino acids, to the parent polypeptide or protein.

In this application, the "functional fragment" of a parent polypeptide or protein generally refers to a peptide or polypeptide (including but not limited to an enzyme) that comprises at least 5 consecutive amino acid residues, at least 10 consecutive amino acid residues, at least 15 consecutive amino acid residues, at least 20 consecutive amino acid residues, at least 25 consecutive amino acid residues, at least 40 consecutive amino acid residues, at least 50 consecutive amino acid residues, at least 60 consecutive amino acid residues, 70 consecutive amino acid residues, 80 consecutive amino acid residues, 90 consecutive amino acid residues, 100 consecutive amino acid residues, 125 consecutive amino acid residues, 150 consecutive amino acid residues, 175 consecutive amino acid residues, 200 consecutive amino acid residues, 250 consecutive amino acid residues, or 350 consecutive amino acid residues, wherein the functional fragment has at least part of the functions of its parent polypeptide or protein. For example, the "functional fragment" of a parent enzyme retains enzymatic activity to a similar extent, to the same extent, or to a greater extent than the parent enzyme.

### DETAILED DESCRIPTION

### Isolated polypeptide, nucleic acid molecule, vector, cell, and catalytic preparation

In one aspect, this application provides an isolated polypeptide. The isolated polypeptide may comprise a catalytically active domain and one or more heptapeptide repeat fragments, the catalytically active domain may comprise an amino acid sequence as set forth in SEQ ID NO: 1. the heptapeptide repeat fragment may comprise an amino acid sequence as set forth in SEQ ID NO: 2, and in the isolated polypeptide, the number of the heptapeptide repeat fragments is not more than 3 (such as, 1, 2, or 3).

For example, a wild-type α1,3 fucosyltransferase (GenBank accession number AAD07710.1) derived from Helicobacter pylori comprises ten heptapeptide repeat fragments (e.g., each of the heptapeptide repeat fragments has the amino acid sequence as set forth in SEQ ID NO: 2). When the C-terminus is truncated (for example, 7-9 heptapeptide repeat fragments are truncated), the isolated polypeptide of this application can be obtained.

In some cases, the catalytically active domain has the amino acid sequence as set forth in SEQ ID NO: 1. In some cases, each of the heptapeptide repeat fragments has the amino acid sequence as set forth in SEQ ID NO: 2.

In the isolated polypeptide of this application, the catalytically active domain may be located at an N-terminus of the heptapeptide repeat fragment. For example, the C-terminus of the catalytically active domain may be directly or indirectly linked to the N-terminus of the heptapeptide repeat fragment.

In the isolated polypeptide of this application, the number of the heptapeptide repeat fragments may be 1. In this case, the catalytically active domain may be located at the N-terminus of the heptapeptide repeat fragment. For example, the C-terminus of the catalytically active domain may be directly or indirectly linked to the N-terminus of the heptapeptide repeat fragment.

For example, the isolated polypeptide of this application may comprise an amino acid sequence as set forth in SEQ ID NO: 3. In some cases, the isolated polypeptide has the amino acid sequence as set forth in SEQ ID NO: 3.

In the isolated polypeptide of this application, the number of the heptapeptide repeat fragments may be 2. For example, two the heptapeptide repeat fragments may be directly linked to each other. For example, the C-terminus of a first heptapeptide repeat fragment may be directly linked (e.g., via a peptide bond) to the N-terminus of a second heptapeptide repeat fragment. For example, in the isolated polypeptide of this application, the C-terminus of the catalytically active domain may be directly linked to the N-terminus of the first heptapeptide repeat fragment, and the C-terminus of the first heptapeptide repeat fragment may be directly linked to the N-terminus of the second heptapeptide repeat fragment.

For example, the isolated polypeptide of this application may comprise an amino acid sequence as set forth in SEQ ID NO: 4. For example, the isolated polypeptide of this application may have the amino acid sequence as set forth in SEQ ID NO: 4.

In the isolated polypeptide of this application, the number of the heptapeptide repeat fragments may be 3. For example, three the heptapeptide repeat fragments may be directly linked in sequence. For example, the C-terminus of a first heptapeptide repeat fragment may be directly linked (e.g., via a peptide bond) to the N-terminus of a second heptapeptide repeat fragment, and the C-terminus of the second heptapeptide repeat fragment may be directly linked (e.g., via a peptide bond) to the N-terminus of a third heptapeptide repeat fragment. For example, in the isolated polypeptide of this application, the C-terminus of the catalytically active domain may be directly linked to the N-terminus of the first heptapeptide repeat fragment, the C-terminus of the first heptapeptide repeat fragment may be directly linked to the N-terminus of the second heptapeptide repeat fragment, and the C-terminus of the second heptapeptide repeat fragment may be directly linked to the N-terminus of the third heptapeptide repeat fragment.

For example, the isolated polypeptide of this application may comprise an amino acid sequence as set forth in SEQ ID NO: 5. For example, the isolated polypeptide of this application may have the amino acid sequence as set forth in SEQ ID NO: 5.

The isolated polypeptide of this application may further comprise a tag sequence, such as an affinity purification tag sequence. The affinity purification tag sequence may include, for example, but be not limited to, polyhistidine tag, Arg tag, FLAG tag, 3xFlag tag, streptavidin tag, nanotag, SBP tag, c-myc tag, S-tag, calmodulin-binding peptide, cellulose-binding domain, chitin-binding domain, GST tag and/or MBP tag.

For example, the isolated polypeptide of this application may comprise an amino acid sequence as set forth in any one of SEQ ID NOs: 3-5 and 8-10. For example, the isolated polypeptide of this application may have the amino acid sequence as set forth in any one of SEQ ID NOs: 3-5 and 8-10.

The isolated polypeptide of this application also comprises functional variants and/or functional fragments thereof. This application further provides one or more isolated nucleic acid molecules that may encode the isolated polypeptide of this application. For example, the nucleic acid molecule encoding the isolated polypeptide of this application may comprise a nucleotide sequence as set forth in any one of SEQ ID NOs: 25-30. In some cases, the nucleic acid sequence encoding the isolated polypeptide of this application is as set forth in any one of SEQ ID NOs: 25-30.

This application further provides a vector (e.g., one or more vectors) which may comprise the nucleic acid sequence of the nucleic acid molecule of this application. For example, the one or more nucleic acid molecules may be contained in one vector or may be contained in multiple vectors. Each vector may comprise one nucleic acid molecule described herein, or may comprise multiple nucleic acid molecules described herein. The vector may be, for example, a plasmid.

This application further provides a cell, which may comprise and/or express the isolated polypeptide of this application, the nucleic acid molecule of this application, and/or the vector of this application. the cell may be, for example, a prokaryotic cell or a eukaryotic cell. For example, the cell may be a bacterium (e.g., E. coli). the cell may also be a eukaryotic microorganism such as a filamentous fungus or a yeast cell, an invertebrate cell, a vertebrate cell (e.g., a mammalian cell such as a CHO cell and a human cell).

This application further provides a catalytic preparation, which may comprise the isolated polypeptide of this application. The catalytic preparation of this application may further comprise the nucleic acid molecule, vector and/or cell of this application. The catalytic preparation may further comprise other catalysts (for example, suitable metal ions, other glycosyltransferases, for example, galactosyltransferases, such as β1,4 galactosyltransferases, glycosidases, such as endoglycosidases, α1,6 fucosidase, such as Alfc).

### Preparation method

This application further provides a method for transferring a fucose and/or a fucose derivative, including: using the isolated polypeptide of this application (e.g., the isolated polypeptide has the amino acid sequence as set forth in any one of SEQ ID NOs: 3-5 and 8-10), the nucleic acid molecule of this application (e.g., the nucleic acid molecule has the amino acid sequence as set forth in any one of SEQ ID NOs: 25-30), the vector of this application, and/or the cell of this application.

This application further provides a use of the isolated polypeptide of this application in the preparation of a protein conjugate.

For example, this application provides a method for preparing a protein conjugate, including: using the isolated polypeptide of this application, the nucleic acid molecule of this application, the vector of this application, the cell of this application, and/or the catalytic preparation of this application.

The preparation method of this application includes: in the presence of the isolated polypeptide of this application and/or the catalytic preparation of this application, contacting a donor Q-Fuc* with a protein having a glycan chain comprising a structure of formula (I) to obtain a protein conjugate, the protein conjugate having a glycan chain comprising a structure of formula (II):

-GlcNAc(Fuc)_{b}-GalX, formula (I);

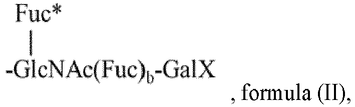

wherein
said GlcNAc is a N-acetylglucosamine;
Fuc is a fucose, and b is 0 or 1;
GalX is an optionally substituted galactose;
the Fuc* comprises a structure of Fuco-MOI, wherein the structure of Fuco is represented by formula (III): 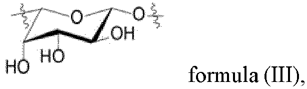 an MOI is a molecule of interest comprising an active substance (AM), the MOI is linked to the left terminus of formula (III), and Q is a ribonucleotide diphosphate.

In certain embodiments, the protein comprises a Fc domain and/or an antigen-binding moiety, the GalX and the GlcNAc are linked via a β-1,4 glycosidic linkage, the Fuc and the GlcNAc are linked via an α-1,6 glycosidic linkage, and the Fuc* and the GlcNAc are linked via an α-1,3 glycosidic linkage.

For example, in the preparation method of this application, the GalX may be an optionally substituted galactose.

For example, the GalX may be an unsubstituted galactose or a substituted galactose. For example, the GalX may be a substituted galactose, and one or more hydroxyls at positions C2, C3, C4 and/or C6 in the galactose may be substituted. In some cases, the GalX may be a substituted galactose, and a hydroxyl at position C2 in the galactose may be substituted.

For example, the GalX may be a monosaccharide. the monosaccharide may, for example, be a molecule that can no longer be simply hydrolyzed into smaller sugars. For example, the monosaccharide may be a monosaccharide derivative but contain only one core monosaccharide backbone. For example, the LacNAc, which contains both Gal and GlcNAc, is not a monosaccharide.

For example, the GalX may be substituted with a substituent Rg', the Rg¹ may be a group selected from the group consisting of: hydrogen, halogen, -NH₂, -SH, -N₃, -COOH, -CN, C₁-C₂₄ alkyl group, C₃-C₂₄ cycloalkyl group, C₂-C₂₄ alkenyl group, C₅-C₂₄ cycloalkenyl group, C₂-C₂₄ alkynyl group, C₇-C₂₄ cycloalkynyl group, C₂-C₂₄ (hetero)aryl group, C₃-C₂₄ alkyl(hetero)aryl group, and C₃-C₂₄ (hetero)arylalkyl group; wherein the alkyl group, cycloalkyl group, alkenyl group, cycloalkenyl group, alkynyl group, cycloalkynyl group, (hetero)aryl group, alkyl(hetero)aryl group and/or (hetero)arylalkyl group may be each independently optionally substituted with one or more substituents Rs'(s), and/or each independently optionally spaced by one or more substituents Rs²(s); each of the Rs¹(s) may be independently a group selected from the group consisting of: halogen, -OH, -NH₂, -SH, -N₃, -COOH, and -CN. Each of the Rs²(s) may be independently a group selected from the group consisting of: -O-, -S-, 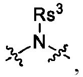 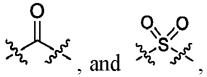 wherein the Rs³ may be a group selected from the group consisting of: hydrogen, C₁-C₂₄ alkyl group, C₂-C₂₄ alkenyl group, C₂-C₂₄ alkynyl group, and C₃-C₂₄ cycloalkyl group, wherein the C₁-C₂₄ alkyl group, C₂-C₂₄ alkenyl group, C₂-C₂₄ alkynyl group, and C₃-C₂₄ cycloalkyl group are optionally substituted.

For example, the GalX may be a C2-substituted galactose, for example, it may be 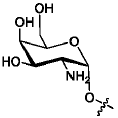 (GalNH₂). For another example, the GalX may be a C2-substituted galactose, for example, it may be

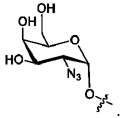

For example, the GalX may be substituted with a substituent 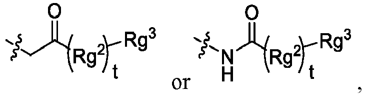 wherein: t may be 0 or 1; the Rg² is a group selected from the group consisting of: C₁-C₂₄ alkylene group, C₃-C₂₄ cycloalkylene group, C₂-C₂₄ alkenylene group, C₅-C₂₄ cycloalkenylene group, C₂-C₂₄ alkynylene group, C₇-C₂₄ cycloalkynylene group, C₂-C₂₄ (hetero)arylene group, C₃-C₂₄ alkyl(hetero)arylene group, and C₃-C₂₄ (hetero)arylalkylene group, wherein the alkylene group, cycloalkylene group, alkenylene group, cycloalkenylene group, alkynylene group, cycloalkynylene group, (hetero)arylene group, alkyl(hetero)arylene group and/or (hetero)arylalkylene group may be each independently optionally substituted with one or more substituents Rs¹, and/or each independently optionally spaced by one or more substituents Rs². The Rg³ is a group selected from the group consisting of: hydrogen, halogen, -OH, -NH₂, -SH, -N₃, -COOH, -CN, C₁-C₂₄ alkyl group, C₃-C₂₄ cycloalkyl group, C₂-C₂₄ alkenyl group, C₅-C₂₄ cycloalkenyl group, C₂-C₂₄ alkynyl group, C₇-C₂₄ cycloalkynyl group, and C₂-C₂₄ (hetero)aryl group; wherein the alkyl group, cycloalkyl group, alkenyl group, cycloalkenyl group, alkynyl group, cycloalkynyl group, and/or (hetero)aryl group are each independently optionally substituted with one or more Rs¹(s). Each of the Rs²(s) may be independently selected from the group consisting of: -O-, -S-, 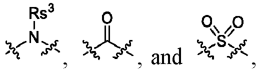 the Rs³ is a group selected from the group consisting of: hydrogen, C₁-C₂₄ alkyl group, C₂-C₂₄ alkenyl group, C₂-C₂₄ alkynyl group, and C₃-C₂₄ cycloalkyl group, wherein the C₁-C₂₄ alkyl group, C₂-C₂₄ alkenyl group, C₂-C₂₄ alkynyl group, and C₃-C₂₄ cycloalkyl group are optionally substituted. Each of the Rs¹(s) may be independently selected from the group consisting of: halogen, -OH, -NH₂, -SH, -N₃, -COOH, and -CN.

For example, the GalX may be a C2-substituted galactose, for example, it may be 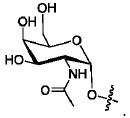 For another example, the GalX may be a C2-substituted galactose, for example, it may be 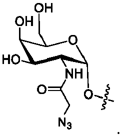 For another example, the GalX may be a C2-substituted galactose, for example, it may be 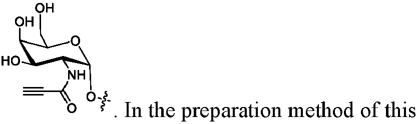 application, the GlcNAc of the formula (I) may be directly linked to an asparagine residue in the protein.

In some cases, in the preparation method of this application, the glycan chain of the protein may comprise a mannose, and the GlcNAc in the formula (I) may be directly linked to the mannose in the glycan chain.

In the preparation method of this application, the protein may comprise a Fc domain, and the glycan chain may be linked to an asparagine residue of the Fc domain.

In the preparation method of this application, the protein may comprise a Fc domain, and the glycan chain may be linked to an asparagine residue of a CH₂ domain in the Fc domain.

For example, in the preparation method of this application, the protein may comprise a Fc domain, and the glycan chain may be linked to position N297 of the Fc domain, wherein the position is determined according to EU index numbering in Kabat.

For example, in the preparation method of this application, the protein may be a Fc domain-containing antibody and/or Fc fusion protein. For example, the protein may be a monoclonal antibody. For example, the protein may be an IgG antibody.

For example, the antibody may be Trastuzumab, its heavy chain may have an amino acid sequence as set forth in SEQ ID NO: 20, and its light chain may have an amino acid sequence as set forth in SEQ ID NO: 19.

For example, the antibody may be Rituximab, its heavy chain may have an amino acid sequence as set forth in SEQ ID NO: 22, and its light chain may have an amino acid sequence as set forth in SEQ ID NO: 21.

For example, the antibody may be Durvalumab, its heavy chain may have an amino acid sequence as set forth in SEQ ID NO: 24, and its light chain may have an amino acid sequence as set forth in SEQ ID NO: 23.

In the preparation method of this application, the Q may be a guanosine diphosphate (GDP), a uridine diphosphate (UDP) and/or a cytidine diphosphate (CDP). For example, the Q may be a guanosine diphosphate (GDP).

In the preparation method of this application, the active substance AM may comprise a biologically active substance, a pharmaceutically active substance, a chemically reactive substance and/or an enzymatically reactive substance. For example, the active substance AM may be a chemically reactive substance and/or an enzymatically reactive substance.

For another example, the active substance AM may be a functional group X₁, and X₁ comprises a functional group capable of participating in a bioorthogonal ligation reaction. For example, the functional group capable of participating in a bioorthogonal ligation reaction may be selected from one or more of the group consisting of: azido, terminal alkynyl, cycloalkynyl, tetrazinyl, 1,2,4-triazinyl, terminal alkenyl, cycloalkenyl, keto, aldehyde, hydroxylamino, mercapto, maleimido and their functional derivatives. For example, the functional group capable of participating in a bioorthogonal ligation reaction may be selected from one or more of the group consisting of:

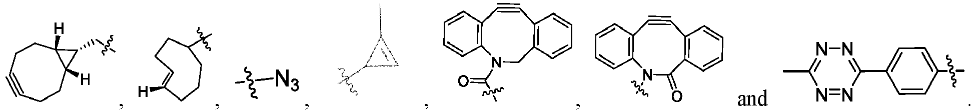

For example, the active substance AM may be a biologically active substance and/or a pharmaceutically active substance P₁. In the preparation method of this application, the active substance AM is a pharmaceutically active substance P₁. For example, the P₁ comprises one or more substances selected from the group consisting of: a cytotoxin, an agonist, an antagonist, an antiviral agent, an antibacterial agent, a radioisotope, a radionuclide, a metal chelator, an oligonucleotide, and a polypeptide. In some cases, the P₁ comprises a cytotoxin.

For example, the P₁ may be selected from one or more of the group consisting of a DNA damaging agent, a topoisomerase inhibitor, an RNA polymerase inhibitor, and a tubulin inhibitor.

For example, the P₁ may comprise a cytotoxin, and the cytotoxin may be selected from one or more of the group consisting of: pyrrolobenzodiazepine and a derivative thereof, auristatin and a derivative thereof, maytansinoids and a derivative thereof, duocarmycin and a derivative thereof, tubulysin and a derivative thereof, enediyene and a derivative thereof, doxorubicin and a derivative thereof, pyrrole-based kinesin spindle protein inhibitor and a derivative thereof, calicheamicin and a derivative thereof, amanitin and a derivative thereof, and camptothecin and a derivative thereof.

For example, the P₁ may comprise a cytotoxin and the cytotoxin may be selected from one or more of the group consisting of: MMAE (Monomethylauristatin E) and DXd (Exatecan derivative).

In the preparation method of this application, the MOI may comprise a linker L. the L may be used for linking the AM to the Fuco. For example, during formation of the conjugate, the L may be located between the AM and the Fuco.

In certain embodiments, the linker L may have a structure of J-(Sp)ₙ, wherein n is 0 or 1, J is a connector, Sp is a spacer, and the J is directly linked to the Fuco. J may function to link the Fuco to the Sp or the AM. For example, the L may consist of the J and the Sp (e.g., n is 1), and the connector J links the Fuco to the Sp. For example, the L may consist of the J (n is 0), and the connector J directly links the Fuco to the AM.

For example, the J may be a structure selected from the group consisting of: 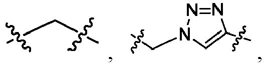 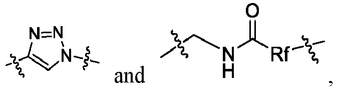 wherein Rf is -CH₂-, -NH- or -O-, wherein the left terminus of the structure may be directly linked to the Fuco.

For example, the J may be 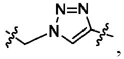 and the left terminus of the structure may be directly linked to the Fuco.

For example, the J may be 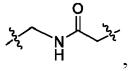 and the left terminus of the structure may be directly linked to the Fuco.

In certain embodiments, the isolated polypeptide of this application has better catalytic activity in catalyzing the transfer of Q-Fuc* comprising the J structure of 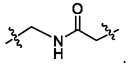 For example, Examples 22 and 23 compare the catalytic efficiency of the isolated polypeptide of this application in catalyzing the Q-Fuc* (e.g., GDP-Fuc*) comprising different J structures.

For example, the spacer Sp described herein may be a structure moiety linking the connector J to the AM.

For example, the spacer Sp described herein may comprise a cleavable moiety. For example, the cleavable moiety may be selected from one or more of the group consisting of: an acid-labile cleavable moiety, a redox-active cleavable moiety, a photoactive cleavable moiety, and a proteolytically cleavable moiety. For example, the cleavable moiety may be selected from one or more of the group consisting of: vc-PAB, GGFG, and disulfide bond.

In certain embodiments, the spacer Sp does not comprise a cleavable moiety. For example, the Sp is uncleavable.

Generally, the spacer Sp can have one or more of the following functions: i) adjusting the distance between the AM and the Fuco; ii) adjusting the hydrophilicity or hydrophobicity of the Fuc* structure; iii) adjusting the rigidity and flexibility of the Fuc* structure; or iv) fragmenting under specific conditions and releasing the active molecule (AM) in the case where the Sp comprises a cleavable moiety.

For example, the example of the Sp structure may be selected from the following structure: 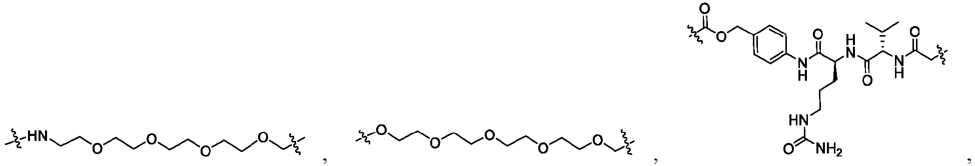 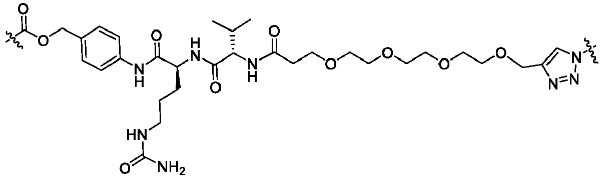 and 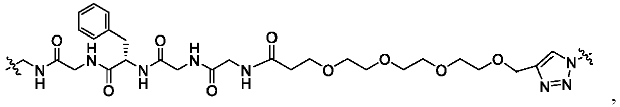 wherein the right terminus of the aforementioned structure may be linked to the J, and the left terminus may be linked to the active molecule AM.

For example, the Q-Fuc* may be a Q-Fuco-L-AM.

For example, in the preparation method described herein, the Q-Fuc* may be a Q-Fuco-J-(Sp)ₙ-AM, wherein n may be 0 or 1. For example, the n may be 0. For another example, the n may be 1.

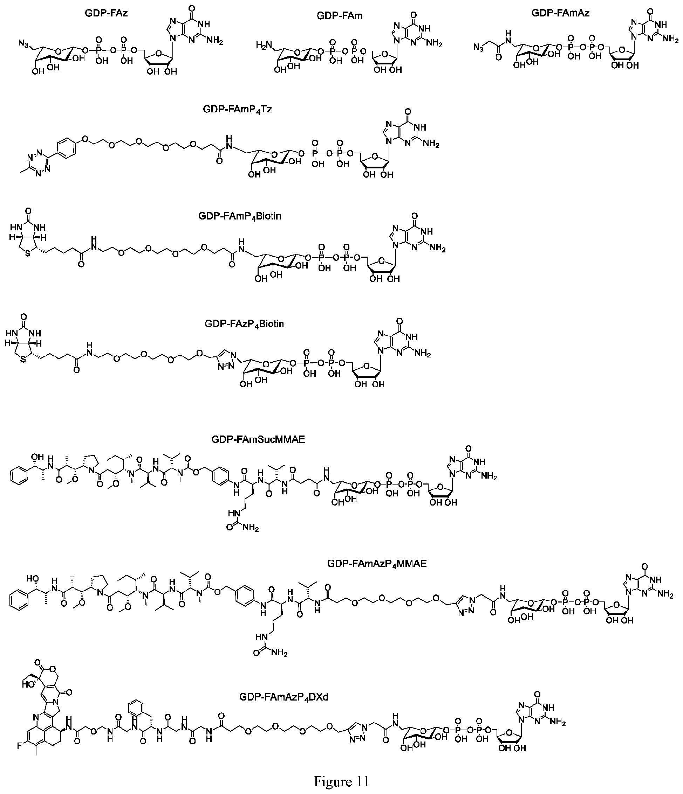

For example, in the preparation method of this application, the Q-Fuc* may be a Q-Fuco-J-(Sp)ₙ-X₁, wherein n may be 0 or 1. For example, n may be 0. For another example, the n may be 1. For example, Figure 11 of this application lists some structure examples of the Q-Fuco-J-(Sp)ₙ-X₁, such as GDP-FAz, GDP-FAmAz, and GDP-FAmP₄Tz. For example, in the preparation method of this application, the Q-Fuc* may be a Q-Fuco-J-(Sp)ₙ-P₁, wherein n may be 0 or 1. For example, the n may be 0. For another example, the n may be 1. For example, Figure 11 of this application lists some structure examples of Q-Fuco-J-(Sp)ₙ-P₁, such as GDP-FAmAzP₄MMAE, GDP-FAmSucMMAE, and GDP-FAmAzP₄DXd.

For example, in the preparation method of this application, the GalX may be selected from one or more of the group consisting of:

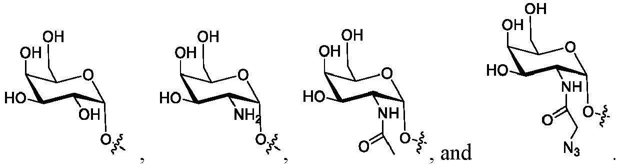

According to the preparation method of this application, the protein conjugate obtained may comprise 1 to 20 structures of formula (II), such as about 2 to 20, about 3 to 20, about 4 to 20, about 5 to 20, about 6 to 20, about 7 to 20, about 8 to 20, about 9 to 20, about 10 to 20, about 11-20, about 12 to 20, about 13 to 20, about 14 to 20, about 15 to 20, about 16 to 20, about 17 to 20, about 18 to 20, about 18 to 20, or about 19 to 20 structures of formula (II). For example, in the preparation method of this application, the protein conjugate may comprise about two or four structures of formula (II).

For example, in the preparation method of this application, the protein conjugate may comprise about four structures of formula (II).

In the preparation method of this application, the structure of the protein conjugate obtained may be represented by formula (IV): 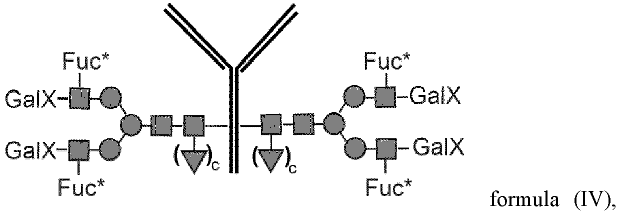 wherein 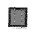 is the N-acetylglucosamine, 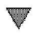 is the fucose, 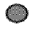 is the mannose, the GalX is the optionally substituted galactose, 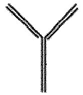 is a Fc domain-containing antibody and/or Fc fusion protein, c is 0 or 1, and the glycan chain is linked to the Fc domain of the Fc domain-containing antibody and/or Fc fusion protein.

In certain embodiments, the method further includes the following step: contacting a Fc domain-containing antibody and/or Fc fusion protein having a G₀(F)_{0,1}, G₁(F)_{0,1} and/or G₂(F)_{0,1} glycoform with a UDP-GalX in the presence of a suitable catalyst to obtain a protein (such as a glycoprotein) having a glycan chain comprising a structure of formula (I), the structure of the protein being represented by formula (V): 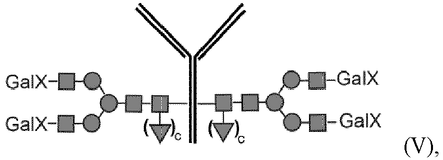 wherein 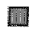 is the N-acetylglucosamine, 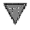 is the fucose, 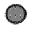 is the mannose, the GalX is the galactose, 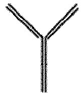 is the Fc domain-containing antibody and/or Fc fusion protein, c is 0 or 1, and the glycan chain is linked to the Fc domain of the Fc domain-containing antibody and/or Fc fusion protein.

For example, the suitable catalyst may be a β1,4 galactosyltransferase or a functional variant thereof. For example, the suitable catalyst may comprise an amino acid sequence as set forth in SEQ ID NO: 16.

For example, therapeutic monoclonal antibodies usually have a conservative N-glycosylation modification on the asparagine at position 297 in the Fc domain (i.e., the position N297 described herein). This modified glycan group is generally composed of a variety of monosaccharides and may have a dual-antenna structure. Typically, more than 90% of the N-glycosylation modifications consist of G₀(F)_{0,1}, G₁(F)_{0,1} and G₂(F)_{0,1} glycoforms. Therefore, the glycoprotein with the structure represented by formula (V) may be obtained through the aforementioned steps. For example, the method of this application may further include the following step: contacting the Fc domain-containing antibody and/or Fc fusion protein having a G₀(F)_{0,1} glycoform with a UDP-GalX in the presence of a suitable catalyst to obtain a glycoprotein having a glycan chain comprising a structure of formula (I), the structure of the glycoprotein being represented by formula (V): 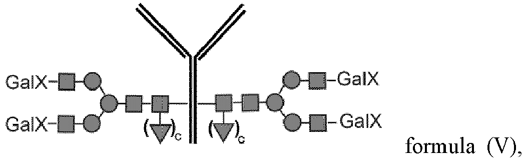 wherein 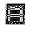 is the N-acetylglucosamine, 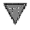 is the fucose, 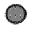 is the mannose, the GalX is the substituted galactose, 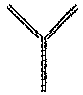 is the Fc domain-containing antibody and/or Fc fusion protein, c is 0 or 1, and the glycan chain is linked to the Fc domain of the Fc domain-containing antibody and/or Fc fusion protein.

For example, the suitable catalyst may be a β1,4 galactosyltransferase or a functional variant thereof. For example, the suitable catalyst may comprise an amino acid sequence as set forth in SEQ ID NO: 16.

In the preparation method described herein, the protein conjugate may comprise two structures of formula (II).

For example, the structure of the protein conjugate may be represented by formula (VI): 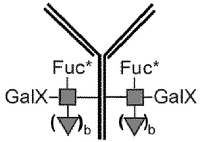 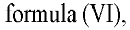 wherein 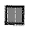 is the N-acetylglucosamine, 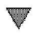 is the fucose, the GalX is the optionally substituted galactose, 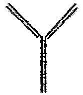 is a Fc domain-containing antibody and/or Fc fusion protein, b is 0 or 1, and the glycan chain is linked to the Fc domain of the Fc domain-containing antibody and/or Fc fusion protein.

For example, the method of this application further includes the following steps: treating the Fc domain-containing antibody and/or Fc fusion protein with an endoglycosidase to obtain the Fc domain-containing antibody and/or Fc fusion protein treated; and contacting the treated Fc domain-containing antibody and/or Fc fusion protein with a UDP-GalX in the presence of a suitable catalyst to obtain a glycoprotein having a glycan chain comprising the structure of formula (I), the structure of the glycoprotein being represented by formula (VII): 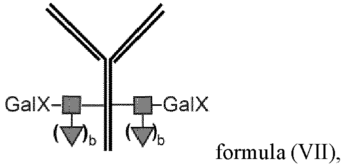 wherein 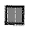 is the N-acetylglucosamine, 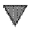 is the fucose, the GalX is the optionally substituted galactose, 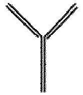 is the Fc domain-containing antibody and/or Fc fusion protein, b is 0 or 1, and the glycan chain is linked to the Fc domain of the Fc domain-containing antibody and/or Fc fusion protein. For example, the suitable catalyst may be a β1,4 galactosyltransferase or a functional variant thereof. For example, the suitable catalyst may comprise an amino acid sequence as set forth in SEQ ID NO: 16. For example, the endoglycosidase may be EndoS, Endo S2, Endo A, Endo F, Endo M, Endo D, Endo H, and/or their functional variants. For example, the endoglycosidase may comprise an amino acid sequence as set forth in SEQ ID NO: 17. For example, the method of this application further includes the following steps: treating the Fc domain-containing antibody and/or Fc fusion protein with an endoglycosidase and an α1,6 fucosidase to obtain the Fc domain-containing antibody and/or Fc fusion protein treated; and contacting the treated Fc domain-containing antibody and/or Fc fusion protein with a UDP-GalX in the presence of the β1,4 galactosyltransferase or a functional variant thereof to obtain a glycoprotein having a glycan chain comprising a structure of formula (I), the structure of the glycoprotein being represented by formula (VII): 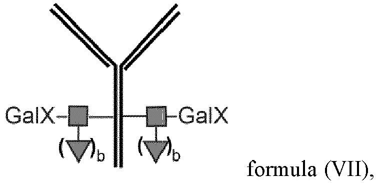 wherein 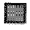 is the N-acetylglucosamine, 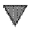 is the fucose, the GalX is the optionally substituted galactose, 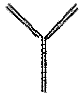 is the Fc domain-containing antibody and/or Fc fusion protein, b is 0, and the glycan chain is linked to the Fc domain of the Fc domain-containing antibody and/or Fc fusion protein. For example, the suitable catalyst may be a β1,4 galactosyltransferase or a functional variant thereof. For example, the suitable catalyst may comprise an amino acid sequence as set forth in SEQ ID NO: 16. For example, the endoglycosidase may be EndoS, Endo S2, Endo A, Endo F, Endo M, Endo D, Endo H, and/or their functional variants. For example, the endoglycosidase may comprise an amino acid sequence as set forth in SEQ ID NO: 17. For example, the α1,6 fucosidase may be BfFucH, Alfc, BKF, and/or their functional variants. For example, the α1,6 fucosidase may comprise an amino acid sequence as set forth in SEQ ID NO: 18.

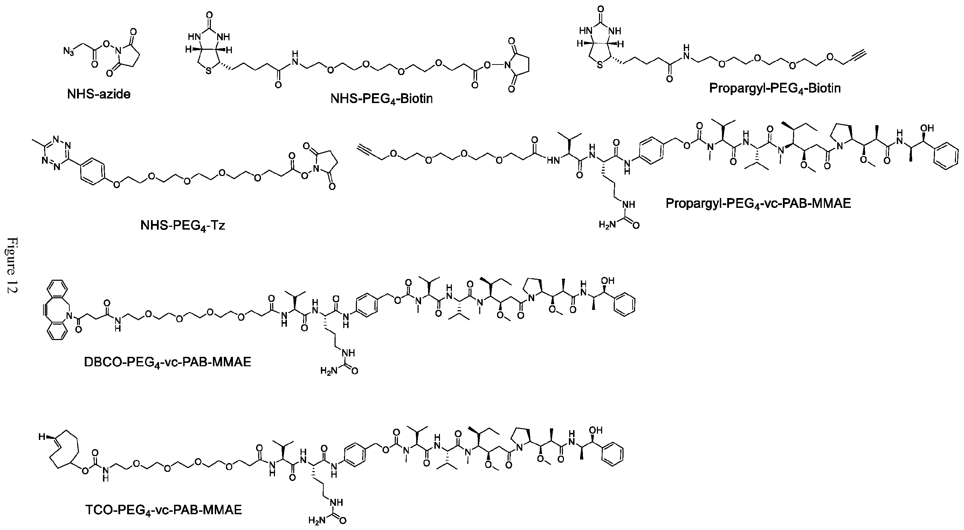

In certain embodiments, the method of this application further includes the following step: reacting the obtained protein conjugate with a Y₁-L'-P₁' to obtain a protein conjugate comprising a biologically active substance and/or a pharmaceutically active substance P₁', wherein the Y₁ is a functional group capable of having a ligation reaction with the X₁, and L' is a linker. For example, the protein conjugate may comprise a functional group X₁ capable of having a ligation reaction with the Y₁. The linker L' may be a structure linking Y₁ to P₁'. For example, P₁' may comprise a toxin molecule. For example, DBCO-PEG₄-vc-PAB-MMAE and TCO-PEG₄-vc-PAB-MMAE shown in Figure 12 are examples of the Y₁-L'-P₁' structure.

One or more protein conjugates or compositions thereof prepared by the method of this application may have a MAR of about 3.2-4.0 or 1.6-2.0.

One or more protein conjugates or compositions thereof prepared by the method of this application may have a MAR of about 3.6-4.0 or 1.8-2.0.

For example, one or more protein conjugates or compositions thereof prepared by the method of this application may have a MAR of about 3.2-4.0.

For example, one or more protein conjugates or compositions thereof prepared by the method of this application may have a MAR of about 3.6-4.0.

For another example, one or more protein conjugates or compositions thereof prepared by the method of this application may have a MAR of about 1.6-2.0.

For another example, one or more protein conjugates or compositions thereof prepared by the method of this application may have a MAR of about 1.8-2.0.

### Protein conjugate, composition and pharmaceutical use

In another aspect, this application further provides a protein conjugate prepared by the method of this application and/or a compositions of multiple protein conjugates.

In another aspect, this application provides a protein conjugate with biological and/or pharmaceutical activity, prepared by using a protein conjugate with chemical and/or enzymatic reactivity, the protein conjugate with chemical and/or enzymatic reactivity being prepared by the method of this application. For example, a protein conjugate with chemical and/or enzymatic reactivity can be prepared by the preparation method of this application, and then the protein conjugate with chemical and/or enzymatic reactivity can be used to prepare the protein conjugate with biological and/or pharmaceutical activity.

For example, the protein conjugate may comprise about four structures of formula (II).

For example, the structure of the protein conjugate obtained may be represented by formula (IV): 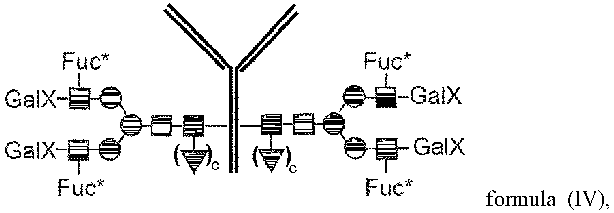 wherein 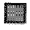 is the N-acetylglucosamine, 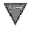 is the fucose, 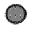 is the mannose, the GalX is the optionally substituted galactose, 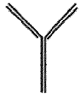 is a Fc domain-containing antibody and/or Fc fusion protein, c is 0 or 1, and the glycan chain is linked to the Fc domain of the Fc domain-containing antibody and/or Fc fusion protein.

In some cases, the protein conjugate may comprise two structures of formula (II).

For example, the structure of the protein conjugate may be represented by formula (V): 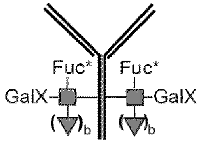 formula (V), wherein 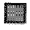 is the N-acetylglucosamine, 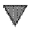 is the fucose, the GalX is the optionally substituted galactose, 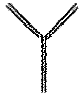 is a Fc domain-containing antibody and/or Fc fusion protein, b is 0 or 1, and the glycan chain is linked to the Fc domain of the Fc domain-containing antibody and/or Fc fusion protein.

This application further provides a composition, which may comprise the isolated polypeptide of this application, the nucleic acid molecule of this application, the vector of this application, the cell of this application, the catalytic preparation of this application, and/or the protein conjugate of this application.

The composition of this application may further comprise one or more pharmaceutically acceptable adjuvants or aids. For example, the adjuvants or aids may include excipients, binders, disintegrants, fillers (diluents), lubricants, glidants (flow enhancers), concentration aids, pigments, sweeteners, preservatives, suspending/dispersing agents, film formers/coatings, flavoring agents and/or printing inks, etc.

The composition of this application may further comprise one or more other active substances, for example, chemically active substances, biologically active substances and/or enzymatically active substances, etc.

In another aspect, this application provides a use of the protein conjugate of this application and/or the composition of this application in the preparation of a medicament.

In another aspect, this application provides a method for preventing, alleviating and/or treating a disease or a disorder. The method may comprise: administering the protein conjugate of this application and/or the composition (e.g., the pharmaceutical composition) of this application to a subject in need.

In another aspect, this application provides a use of the protein conjugate of this application and/or the composition of this application in the preparation of a medicament, and the medicament may be used for preventing, alleviating and/or treating a disease or a disorder.

Without wishing to be bound by any particular theory, the following examples are only for illustrating the polypeptide of this application and its preparation method and use, but not intended to limit the scope of the invention of this application.

### Examples

### Material and method

### Molecular weight mass spectrometry analysis of proteins

Proteins were subjected to mass analysis on an Xevo G2-XS QTOF MS System (Waters Corporation) equipped with an electrospray ionization (ESI) source in conjunction with an Acuqity UPLC I-Class plus system (Waters Corporation). The purified proteins were subjected to separation and desalting on a Waters ACQUITY UPLC Protein BEH C4 Column (300 Å, 1.7 µm, 2.1 mm x 100 mm). Mobile phase A was 0.1% formic acid in water and mobile phase B was acetonitrile with 0.1% formic acid in water at a flow rate of 0.2 mL/min. Data were analyzed using Waters Unify software (version 1.9.4, Waters Corporation).

### Cloning, expression and purification of HpFT (Helicobacter pylori α-1,3-Fucosyltransferase) variants

The cloning, expression and purification of HpFT-4HR (comprising a catalytically active domain, four heptapeptide repeat sequences and a C-terminus fused His tag and having an amino acid sequence as set forth in SEQ ID NO: 11) were performed according to the reported procedure by Wu P. et. al (Proc. Natl. Acad. Sci. USA 2009, 106, 16096). The nucleic acid sequences of HpFT-6HR (comprising a catalytically active domain, six heptapeptide repeat sequences and a C-terminus fused His tag and having an amino acid sequence as set forth in SEQ ID NO: 12), HpFT-3HR (comprising a catalytically active domain, three heptapeptide repeat sequences and a C-terminus fused His tag and having an amino acid sequence as set forth in SEQ ID NO: 10), HpFT-2HR (comprising a catalytically active domain, two heptapeptide repeat sequences and a C-terminus fused His tag and having an amino acid sequence as set forth in SEQ ID NO: 9), HpFT-1HR (comprising a catalytically active domain, a heptapeptide repeat sequence and a C-terminus fused His tag and having an amino acid sequence as set forth in SEQ ID NO: 8), HpFT-0HR (comprising a catalytically active domain and a C-terminus fused His tag and having an amino acid sequence as set forth in SEQ ID NO: 7), HpFT-4HR (M4) (comprising four site mutants S46F, A128N, H129E and Y132I in its catalytically active domain, four heptapeptide repeat sequences and a C-terminus fused His tag, and having an amino acid sequence as set forth in SEQ ID NO: 13), and HpFT-4HR (M7) (comprising seven site mutants S46F, A128N, H129E, Y132I, Y200N, E341D and V369A in its catalytic active region, four heptapeptide repeat sequences and a C-terminus fused His tag, and having an amino acid sequence as set forth in SEQ ID NO: 14) were encoded through gene synthesis, and the nucleic acid sequences were inserted into the pET24b vector (Nanjing GenScript) through the NdeI and BamHI restriction sites. *E*. *coli* BL21 (DE3) was transformed using the constructed recombinant plasmid. The transformed recombinant bacteria were cultured in LB medium supplemented with 50 µg/mL kanamycin at 37 °C until OD600 reached 0.6-0.8, IPTG (isopropyl-β-D-thiogalactopyranoside) was then added to a final concentration of 0.2 mM, and then the bacteria were further incubated at 25 °C, 200 rpm for 16 h for induced protein expression. The induced bacteria were centrifuged and suspended in lysis buffer (25 mM Tris-HCl, pH 7.5, 500 mM NaCl, 20 mM imidazole, 1 mM PMSF (phenylmethylsulfonyl fluoride). The suspended cells were lysed by sonication and then purified with Ni-NTA packing (GE Healthcare). The major fractions with a purity greater than 90% were collected and then dialyzed into storage buffer (25 mM Tris, pH 7.5, 150 mM NaCl, 5% glycerol).

### Cloning, expression and purification of BGalT1(Y289L) (Bovine β-1,4-galactosyltransferase I with Y289L mutant), EndoS (Streptococcus pyogenes endoglycosidase S), Alfc (Lactobacillus casei α-1,6-fucosidase Alfc), and HFT6 (Human fucosyltransferase-6)

The cloning, expression and purification of BGalT1(Y289L) (SEQ ID NO: 16), EndoS (SEQ ID NO: 17), Alfc (SEQ ID NO: 18) and HFT6 (SEQ ID NO: 15) were performed according to the reported procedure by Qasba, P. K et al. (J. Biol. Chem. 2002, 277 (23): 20833-20839; Prot. Expr. Fur. 2003, 30, 219.), by Collin, M. et al. (EMBO J. 2001, 20, 3046; Infect. Immun. 2001, 69, 7187), by Wang L., et al. (Methods Mol. Biol. 2018, 19, 367), and by Moremen K.W et al. (Nat Chem. Biol. 2018, 14, 156), respectively.

### Example 1 Synthesis of GDP-FAz

GDP-FAz was synthesized according to the reported procedure (Wu P., et al., Proc. Natl. Acad. Sci. USA 2009, 106, 16096), and purified through a Bio-Gel P-2 Gel column (Biorad) using 50mM ammonium bicarbonate solution as an eluent.

HRMS (ESI-) calcd for C₁₆H₂₄N₈O₁₅P₂(M-H⁺) 629.0764, found 629.0785.

### Example 2 Synthesis of GDP-FAm

To a solution of 100 mg GDP-FAz in 8.75 mL MeOH/H₂O (1:1.5), 5 mg Pd/C was added. The air atmosphere was changed to H₂ in vacuum, and the system pressure was kept at 0.28 MPa. The reaction was allowed for stirring for 4 h and filtered. Concentration and lyophilization give the product as a white solid (84.8 mg, yield 88%). HRMS (ESI-) calcd for C₁₆H₂₆N₆O₁₅P₂ (M-H⁺) 603.0859, found 603.0874. 1H NMR (400 MHz, D2O) δ 8.10 (s, 1H), 5.92 (d, J = 6.1 Hz, 1H), 4.97 (t, J = 7.6, 1H), 4.76-4.73 (m, 1H), 4.51 (dd, J = 5.2, 3.4 Hz, 1H), 4.37-4.34 (m, 1H), 4.23-4.21 (m, 2H), 3.96 (dd, J = 9.6, 2.4 Hz, 1H), 3.92-3.91 (m, 1H), 3.70 (dd, J = 10.0, 3.3 Hz, 1H), 3.63 (dd, J = 10.0, 7.6 Hz, 1H), 3.31 (dd, J = 13.4, 9.6 Hz, 1H), 3.24 (dd, J = 13.4, 3.1 Hz, 1H).

### Example 3 Synthesis of GDP-FAmAz

To a solution of 600 µL GDP-FAm (100 mM in H₂O) were added 200 µL NaHCO₃ (200 mM in H₂O), 780 µL THF (Tetrahydrofuran), and 220 µL NHS-azide (Xi'an Dianhua Biotechnology Co., Ltd) (100 mM in THF) in sequence. The reaction was allowed for stirring at r.t. overnight and monitored by TLC. The crude product was further purified through a Prep-HPLC system to give the product as a white solid (8.7 mg, 63%). HRMS (ESI-) calcd for C₁₈H₂₇N₉O₁₆P₂ (M-H⁺) 686.0978, found 686.1002. ¹H NMR (400 MHz, D₂O): δ 8.10 (s, 1H), 5.92 (d, J = 6.0 Hz, 1H), 4.92 (t, J = 7.9 Hz, 1H), 4.78-4.76 (m, 1H), 4.52 (dd, J = 5.2, 3.4 Hz, 1H), 4.35-4.34 (m, 1H), 4.23-4.21 (m, 2H), 4.00 (s, 2H), 3.87 (d, J = 3.2 Hz, 1H), 3.71 (dd, J = 8.8, 3.9 Hz, 1H), 3.66 (dd, J = 10.0, 3.3 Hz, 1H), 3.62-3.57 (m, 2H), 3.32 (dd, J = 14.0, 8.6 Hz, 1H).

### Example 4 Synthesis of GDP-FAmP₄Biotin

To a solution of 500 µL GDP-FAm (100 mM in H₂O) were added 500 µL NaHCO₃ (200 mM in H₂O), 1.95 mL THF, and 550 µL NHS-PEG4-Biotin (Xi'an Dianhua Biotechnology Co., Ltd) (100 mM in THF) in sequence. The reaction was allowed for stirring at r.t. for 4 h and monitored by TLC. The crude product was further purified through a Prep-HPLC system to give the product as a white solid (20.5 mg, 38%). HRMS (ESI-) calcd for C₃₇H₆₁N₉O₂₂P₂S (M-H⁺) 1076.3054, found 1076.3068. ¹H NMR (400 MHz, D₂O) δ 8.12 (s, 1H), 5.92 (d, J = 6.1 Hz, 1H), 4.93 (t, J = 7.8 Hz, 1H), 4.78-4.77 (m, 1H), 4.59 (dd, J = 7.9, 4.6 Hz, 1H), 4.53 (dd, J = 5.2, 3.4 Hz, 1H), 4.39 (dd, J = 7.9, 4.4 Hz, 1H), 4.35-4.34 (m, 1H), 4.22 (dd, J = 5.4, 3.4 Hz, 2H), 3.87 (d, J = 3.1 Hz, 1H), 3.76 (t, J = 6.3 Hz, 2H), 3.69-3.66 (m, 14H), 3.63-3.60 (m, 3H), 3.59-3.56 (m, 1H), 3.38 (t, J = 5.3 Hz, 2H), 3.32-3.26 (m, 2H), 2.97 (dd, J = 13.1, 5.0 Hz, 1H), 2.77 (d, J = 13.0 Hz, 1H), 2.56 (t, J = 6.2 Hz, 2H), 2.26 (t, J = 7.3 Hz, 2H), 1.74-1.51 (m, 4H), 1.42-1.34 (m, 2H).

### Example 5 Synthesis of GDP-FAzP₄Biotin

To a solution of 400 µL GDP-FAz (50 mM in H₂O) were added 400 µL CuSO₄/BTTP (5 mM/10 mM in H₂O), 210 µL propargyl-PEG₄-Biotin (Xi'an Dianhua Biotechnology Co., Ltd) (100 mM in MeOH), and 40 µL sodium ascorbate (250 mM in H₂O) in sequence. The reaction was allowed for stirring at r.t. for 5 h and monitored by TLC. Then, 2 mM BCS (2,9-dimethyl-4,7-diphenyl-1,10-phenanthroline disulfonate disodium salt) was added to quench the reaction. The crude product was further purified through a Prep-HPLC system to give the product as a white solid (14.4 mg, 60%). HRMS (ESI-) calcd for C₃₇H₅₉N₁₁O₂₁P₂S (M-H⁺) 1086.3010, found 1086.3040. ¹H NMR (400 MHz, D₂O) δ 8.15 (s, 1H), 8.11 (s, 1H), 5.89 (d, J = 6.0 Hz, 1H), 4.94-4.90 (m, 1H), 4.77-4.76 (m, 1H), 4.73-4.68 (m, 1H), 4.65 (d, J = 3.1 Hz, 2H), 4.62-4.59 (m, 1H), 4.58-4.56 (m, 1H), 4.53-4.51 (m, 1H), 4.38 (dd, J = 8.0, 4.4 Hz, 1H), 4.34-4.31 (m, 1H), 4.25-4.16 (m, 2H), 4.05-4.02 (m, 1H), 3.82 (s, 1H), 3.72-3.65 (m, 14H), 3.61 (t, J = 5.3 Hz, 2H), 3.37 (t, J = 5.2 Hz, 2H), 3.30-3.25 (m, 1H), 2.96 (dd, J = 13.1, 5.0 Hz, 1H), 2.77-2.72 (m, 1H), 2.24 (t, J = 7.3 Hz, 2H), 1.74-1.49 (m, 4H), 1.40-1.32 (m, 2H).

### Example 6 Synthesis of GDP-FAmP₄Tz

To a solution of 200 µL GDP-FAm (100 mM in H₂O) were added 200 µL NaHCO₃ (200 mM in H₂O), 780 µL THF, and 220 µL NHS-PEG₄-Tz (100 mM in THF) in sequence. The reaction was allowed for stirring at r.t. for 4 h and monitored by TLC. The crude product was further purified through a Prep-HPLC system to give the product as a pink solid (9.9 mg, 48%). HRMS (ESI-) calcd for C₃₆H₅₂N₁₀O₂₁P₂ (M-H⁺) 1021.2711, found 1021.2725. ¹H NMR (400 MHz, D₂O) δ 8.17-8.13 (m, 2H), 8.00 (s, 1H), 7.06-7.02 (m, 2H), 5.76 (d, J = 5.6 Hz, 1H), 4.91 (t, J = 7.7 Hz, 1H), 4.67 (t, J = 5.4 Hz, 1H), 4.49 (dd, J = 5.1, 3.7 Hz, 1H), 4.30-4.28 (m, 1H), 4.27-4.25 (m, 2H), 4.21-4.19 (m, 2H), 3.95-3.93 (m, 2H), 3.84-3.83 (m, 1H), 3.80-3.78 (m, 2H), 3.74-3.71 (m, 2H), 3.70-3.57 (m, 13H), 3.51 (dd, J = 14.1, 4.2 Hz, 1H), 3.24 (dd, J = 14.0, 8.6 Hz, 1H), 3.00 (s, 3H), 2.49 (t, J = 6.3 Hz, 2H).

### Example 7 Synthesis of GDP-FAmSucMMAE

GDP-FAmSucMMAE was synthesized according to the route listed below:

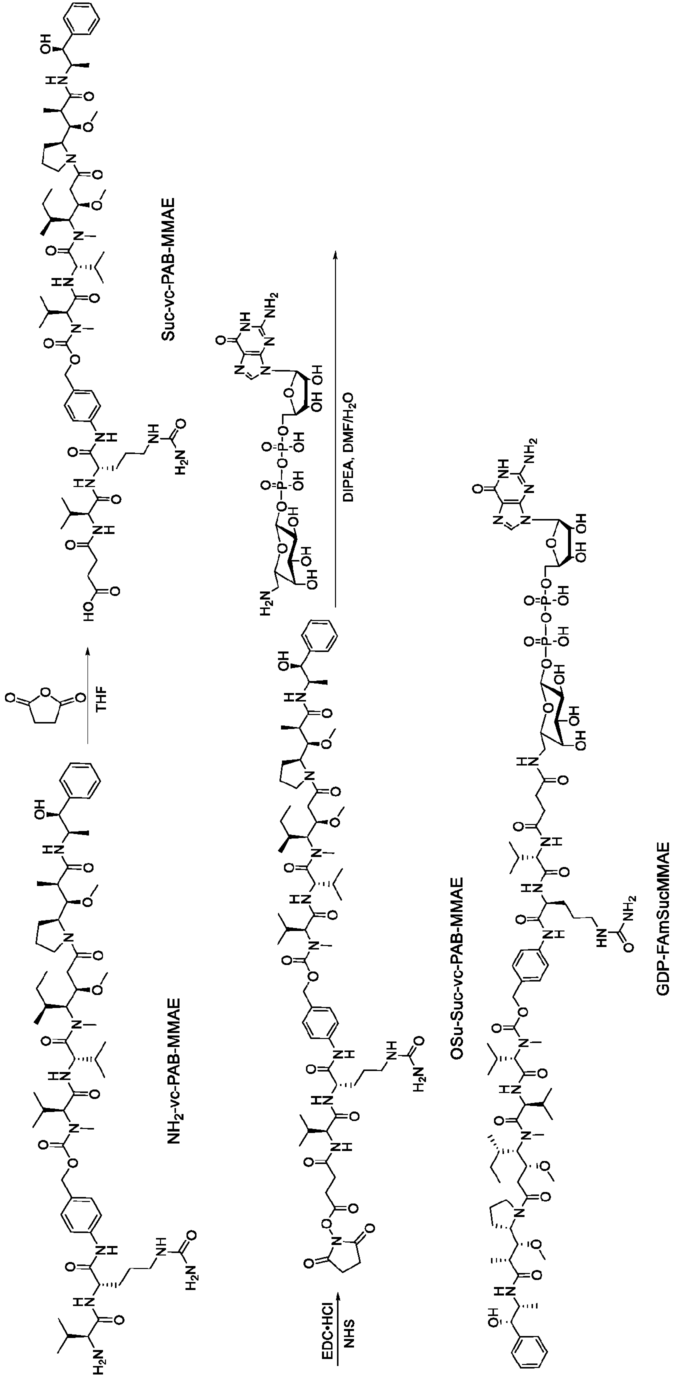

NH₂-vc-PAB-MMAE was synthesized according to the reported procedure (Tang, F., et al. Org. Biomol. Chem. 2016, 14, 9501).

Acid-Suc-vc-PAB-MMAE. Succinic anhydride (120 mg, 1.12 mmol) and NH₂-vc-PAB-MMAE (833 mg, 0.74 mmol) were dissolved in a solution of DMF (N,N-dimethylformamide) (15 mL) and THF (15 mL). The mixture was stirred at r.t. for 5 h and monitored by TLC. The crude product was further purified through a Prep-HPLC system to give the product as a white solid (683 mg, 75%). HRMS (ESI-) calcd for C₆₂H₉₈N₁₀O₁₅ (M-H⁺) 1221.7140, found 1221.7146.

OSu-Suc-vc-PAB-MMAE. Acid-Suc-vc-PAB-MMAE (833 mg, 0.74 mmol) and NHS (N-hydroxysuccinimide) (644 mg, 5.596 mmol) were dissolved in a solution of DMF (15 mL) and THF (15 mL) and EDC•HCl (1-ethyl-(3-dimethylaminopropyl) carbodiimide hydrochloride) (1284 mg, 6.698 mmol) was then added by stirring. The reaction was allowed for stirring at r.t. for 3 h and monitored by TLC. The crude product was further purified through a Prep-HPLC system to give the product as a white solid (565 mg, 77%). HRMS (ESI+) calcd for C₆₆H₁₀₁N₁₁O₁₇ (M+Na⁺) 1342.7269, found 1342.7283.

GDP-FAmSucMMAE. OSu-Suc-vc-PAB-MMAE (346 mg, 0.262 mmol) was dissolved in 12 mL DMF, and 400 uL DIPEA (N,N-diisopropylethylamine) and GDP-FAm (190 mg, 0.315 mmol) (in 30 mL H₂O) were then added respectively by stirring. The reaction was allowed for stirring at r.t. for 5 h and monitored by TLC. The crude product was further purified through a Prep-HPLC system to give the product as a white solid (104.3 mg, 22%). HRMS (ESI-) calcd for C₇₈H₁₂₂N₁₆O₂₉P₂ ((M-2H+)/2) 903.3947, found 903.3959. ¹H NMR (400 MHz, D₂O) δ 8.16 (s, 1H), 7.48-7.40 (m, 3H), 7.37-7.28 (m, 5H), 7.22-7.14 (m, 1H), 5.9 (d, J = 6.0 Hz, 1H), 5.30-5.14 (m, 1H), 5.06-5.00 (m, 1H), 4.76-4.68 (m, 2H), 4.63-4.59 (m, 1H), 4.51-4.49 (m, 1H), 4.46-4.30 (m, 4H), 4.21-4.04 (m, 6H), 3.76-3.63 (m, 2H), 3.57-3.53 (m, 2H), 3.45-3.14 (m, 14H), 3.11-3.05 (m, 4H), 2.94-2.90 (m, 3H), 2.66-2.44 (m, 6H), 2.16-2.01 (m, 3H), 1.94-1.76 (m, 4H), 1.68-1.47 (m, 4H), 1.36-1.14 (m, 8H), 1.06 (d, J = 6.6 Hz, 2H), 0.97-0.89 (m, 10H), 0.85-0.77 (m, 10H), 0.66 (t, J = 7.8 Hz, 2H), 0.48 (d, J = 6.6 Hz, 1H).

### Example 8 Synthesis of GDP-FAmAzP₄MMAE

To a solution of 200 µL GDP-FAmAz (50 mM in H₂O) were added 200 µL CuSO₄/BTTP (5 mM/10 mM in H₂O), 210 µL propargyl-PEG₄-vc-PAB-MMAE (Levena Biopharma) (50 mM in MeOH), and 20 µL sodium ascorbate (250 mM in H₂O) in sequence. The reaction was allowed for stirring at r.t. for 5 h and monitored by TLC. Then, 2 mM BCS was added to quench the reaction. The crude product was further purified through a Prep-HPLC system to give the product as a white solid (16.8 mg, 82%). HRMS (ESI+) calcd for C₈₈H₁₃₉N₁₉O₃₃P₂ ((M+2Na⁺)/2) 1048.9521, found 1048.9533.

### Example 9 Synthesis of GDP-FAmAzP₄DXd

GDP-FAmAzP₄DXd was synthesized according to the route listed below:

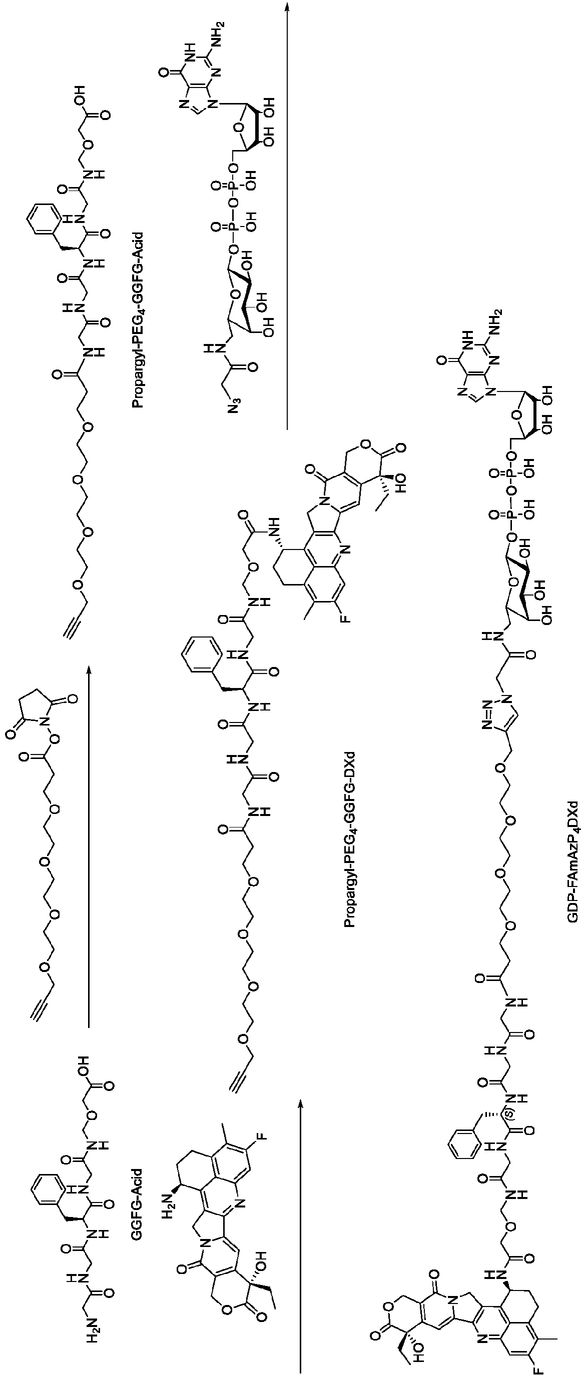

GGFG-Acid was synthesized according to the reference patent (Yamaguchi, T., et al., EP3677589A1).

Propargyl-PEG₄-GGFG-Acid. To a solution of GGFG-Acid (98.4 mg, 0.23 mmol) in 5 ml DMF were added 0.2 mL DIPEA (N,N-diisopropylethylamine) and propargyl-PEG₄-OSu (99.6 mg, 0.28 mmol). The mixture was stirred at r.t. overnight and monitored by TLC. The crude product was further purified through a Prep-HPLC system to give the product as a white solid (114.8 mg, 75%). HRMS (ESI-) calcd for C₃₀H₄₃N₅O₁₂ (M-H⁺) 664.2835, found 664.2808.

Propargyl-PEG₄-GGFG-DXd. To a solution of propargyl-PEG₄-GGFG Acid (66.6 mg, 0.1 mmol) in 5 mL DMF were added 0.1 mL DIPEA, Exatecan (43.5 mg, 0.1 mmol) and PyBOP (benzotriazol-1-yl-oxytripyrrolidinylphosphonium hexafluorophosphate) (104.9 mg, 0.2 mmol). The reaction was allowed for stirring at r.t. for 2 h and monitored by TLC. The crude product was further purified through a Prep-HPLC system to give the product as a light-yellow solid (70.6 mg, 65%). HRMS (ESI+) calcd for C₅₄H₆₃FN₈O₁₅ (M+Na⁺) 1105.4289, found 1105.4255.

GDP-FAmAzP₄DXd. To a solution of 200 µL GDP-FAmAz (50 mM in H₂O) were added 200 µL CuSO₄/BTTP (5 mM/10 mM in H2O), 210 µL propargyl-PEG₄-GGFG-DXd (50 mM in MeOH), and 20 µL sodium ascorbate (250 mM in H2O) in sequence. The reaction was allowed for stirring at r.t. for 5 h and monitored by TLC. Then, 2 mM BCS was added to quench the reaction. The crude product was further purified through a Prep-HPLC system to give the product as a white solid (12.1 mg, 68%). HRMS (ESI-) calcd for C₇₂H₉₀FN₁₇O₃₁P₂ ((M-2H⁺)/2) 883.7651, found 883.7651. ¹H NMR (400 MHz, D₂O) δ 7.97 (s, 2H), 7.21 (s, 1H), 7.11-7.03 (m, 4H), 6.86 (d, J = 7.0 Hz, 2H), 5.68 (d, J = 5.7 Hz, 1H), 5.59-5.55 (m, 1H), 5.45-5.40 (m, 1H), 5.29-5.25 (m, 1H), 5.20 (s, 1H), 4.95-4.91 (m, 2H), 4.70-4.53 (m, 7H), 4.46 (t, J = 4.1 Hz, 1H), 4.37-4.31 (m, 2H), 4.26-4.17 (m, 4H), 3.86-3.57 (m, 26H), 3.34-3.28 (m, 1H), 3.10-3.06 (m, 1H), 2.97-2.88 (m, 1H), 2.78-2.73 (m, 1H), 2.56-2.50 (m, 3H), 2.39-2.29 (m, 1H), 2.09 (s, 3H), 1.93-1.82 (m, 2H), 0.94 (t, J = 7.3, 3H).

### Example 10 Preparation of Trastuzumab-G₂F

To 50 mM Tris-HCl (pH 7.0) buffer were added Trastuzumab (8 mg/mL final), UDP-galactose (5 mM final), MnCl₂ (5 mM final), and BGalT1(Y289L) (0.5 mg/mL final) in sequence, and the resulting mixed solution was allowed to react at 30 °C for 48 h. Then, the reaction solution was purified with protein A resin (Genescript) to give the modified antibody of interest. Mass spectral analysis showed the major peak of trastuzumab-G₂F (found as 148712 Da, >90%).

The light chain of Trastuzumab has an amino acid sequence as set forth in SEQ ID NO: 19, and the heavy chain has an amino acid sequence as set forth in SEQ ID NO: 20.

### Example 11 Preparation of Durvalumab-G₂F

To 50 mM Tris-HCl (pH 7.0) buffer were added Durvalumab (8 mg/mL final), UDP-galactose (5 mM final), MnCl₂ (5 mM final), and BGalT1(Y289L) (0.5 mg/mL final) in sequence, and the resulting mixed solution was allowed to react at 30 °C for 48 h. Then, the reaction solution was purified with protein A resin (Genescript) to give the modified antibody of interest. Mass spectral analysis showed the major peak of Durvalumab-G₂F (found as 149618 Da, >90%).

The light chain of Durvalumab has an amino acid sequence as set forth in SEQ ID NO: 23, and the heavy chain has an amino acid sequence as set forth in SEQ ID NO: 24.

### Example 12 Preparation of Trastuzumab-(Fucα1,6)(Galβ1,4)GlcNAc

First, to 50 mM Tris-HCl (pH 7.0) buffer were added Trastuzumab (5 mg/mL final) and EndoS (0.05 mg/mL final) in sequence, and the resulting mixed solution was allowed to react at 37 °C for 1 h. Then, to the reaction solution were added UDP-galactose (5 mM final), MnCl₂ (5 mM final), and BGalT1(Y289L) (0.5 mg/mL, final) in sequence, and the resulting mixed solution was allowed to further react at 30 °C for 72 h. Then, the reaction solution was purified with protein A resin (Genescript) to give the modified antibody of interest. Mass spectral analysis showed the major peak of Trastuzumab-(Fucα1,6)(Galβ1,4)GlcNAc (146193 Da, >90%).

### Example 13 Preparation of Trastuzumab-(Fucα1,6)(GalNAzβ1,4)GlcNAc

First, to 50 mM Tris-HCl (pH 7.0) buffer were added Trastuzumab (5 mg/mL final) and EndoS (0.05 mg/mL final) in sequence, and the resulting mixed solution was allowed to react at 37 °C for 1 h. Then, to the reaction solution were added UDP-GalNAz (5 mM final), MnCl₂ (5 mM final), and BGalT1(Y289L) (0.5 mg/mL final) in sequence, and the resulting mixed solution was allowed to further react at 30 °C for 24 h. Then, the reaction solution was purified with protein A resin to give the modified antibody of interest. Mass spectral analysis showed the major peak of Trastuzumab-(Fucα1,6)(GalNAzβ1,4)GlcNAc (146373 Da, >90%).

### Example 14 Preparation of Trastuzumab-(GalNAzβ1,4)GlcNAc

First, to 50 mM Tris-HCl (pH 7.0) buffer were added Trastuzumab (5 mg/mL final), EndoS (0.05 mg/mL final) and Alfc (1.5 mg/mL final) in sequence, and the resulting mixed solution was allowed to react at 37 °C for 24 h. Then, to the reaction solution were added UDP-GalNAz (5 mM final), MnCl₂ (5 mM final), and BGalT1(Y289L) (0.5 mg/mL, final) in sequence, and the resulting mixed solution was allowed to further react at 30 °C for 16 h. Then, the reaction solution was purified with protein A resin to give the modified antibody of interest. Mass spectral analysis showed the major peak of Trastuzumab-(GalNAzβ1,4)GlcNAc (146077 Da, >90%).

### Example 15 Preparation of 15 Durvalumab-(GalNAzβ1,4)GlcNAc

First, to 50 mM Tris-HCl (pH 7.0) buffer were added Durvalumab (5 mg/mL final), EndoS (0.05 mg/mL final) and Alfc (1.5 mg/mL final) in sequence, and the resulting mixed solution was allowed to react at 37 °C for 24 h. Then, to the reaction solution were added UDP-GalNAz (5 mM final), MnCl₂ (5 mM final), and BGalT1(Y289L) (0.5 mg/mL final) in sequence, and the resulting mixed solution was allowed to further react at 30 °C for 16 h. Then, the reaction solution was purified with protein A resin to give the modified antibody of interest. Mass spectral analysis showed the major peak of Durvalumab-(GalNAzβ1,4)GlcNAc (146973 Da, >90%).

### Example 16 Preparation of Trastuzumab-(Galβ1,4)GlNAc

First, to 50 mM Tris-HCl (pH 7.0) buffer were added Trastuzumab (5 mg/mL final), EndoS (0.05 mg/mL final) and Alfc (1.5 mg/mL final) in sequence, and the resulting mixed solution was allowed to react at 37 °C for 24 h. Then, to the reaction solution were added UDP-galactose (5 mM final), MnCl₂ (5 mM final), and BGalT1(Y289L) (0.5 mg/mL final) in sequence, and the resulting mixed solution was allowed to further react at 30 °C for 24 h. Then, the reaction solution was purified with protein A resin to give the modified antibody of interest. Mass spectral analysis showed the major peak of Trastuzumab-(Galβ1,4)GlcNAc (145909 Da, >90%).

### Example 17 Comparison of the reaction efficiency of different HpFTs in transferring GDP-FAmP₄Biotin to Trastuzumab-G₂F

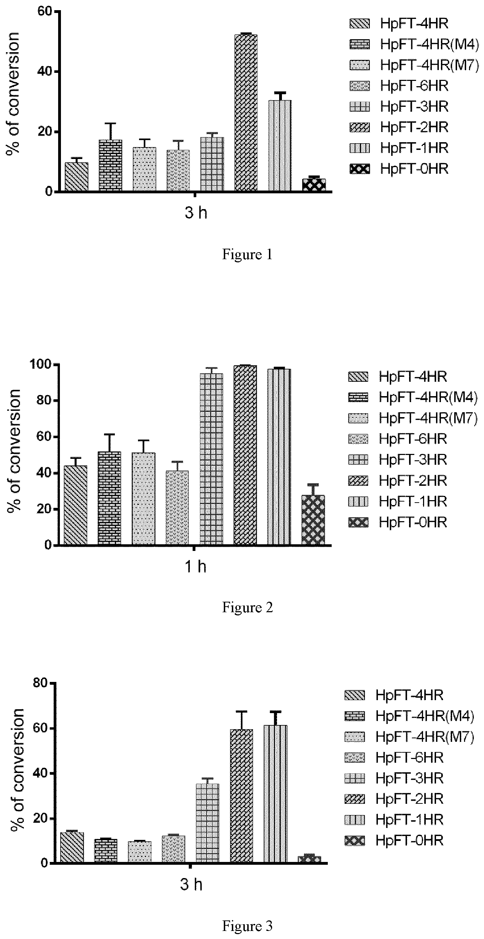

To 40 mM Tris-HCl (pH 7.5) buffer were added Trastuzumab-G₂F (2 mg/mL final), GDP-FAmP₄Biotin (1 mM final), MgCl₂ (20 mM final), and different HpFT enzymes (HpFT-4HR, HpFT-4HR(M4), HpFT-4HR(M7), HpFT-6HR, HpFT-3HR, HpFT-2HR, HpFT-1HR, or HpFT-0HR) (0.5 mg/mL final) in sequence, and the resulting mixed solution was allowed to react at 30 °C for 3 h. Three parallels were set up for each group. After the reaction time point was reached, to the reaction solution was added 10mM LacNAc (N-acetyl-D-lactosamine), and the reaction was terminated by a desalting column (Zeba^{™} Spin Desalting Columns/2 ml/40K, Thermo Fisher Scientific), and finally mass spectrometry was carried out and % of conversion was calculated. % of conversion = (MAR0 peak intensity*0+MAR1peak intensity*1+MAR2 peak intensity*2+MAR3 peak intensity*3+MAR4 peak intensity*4)/4* (MAR0 peak intensity+MAR1 peak intensity+MAR2 peak intensity+MAR3 peak intensity+MAR4 peak intensity)* 100 %. The results are shown in Figure 1. It can be seen that HpFT-3HR, HpFT-2HR, and HpFT-1HR all have significantly improved catalytic activity, as compared with HpFT-4HR. Among them, HpFT-2HR has the highest catalytic activity.

### Example 18 Comparison of the reaction efficiency of different HpFTs in transferring GDP-FAmP₄Biotin to Trastuzumab-(Fucα1,6)(Galβ1,4)GlcNAc

To 40 mM Tris-HCl (pH 7.5) buffer were added Trastuzumab-(Fucα1,6)(Galβ1,4)GlcNAc (2 mg/mL final), GDP-FAmP₄Biotin (1 mM final), MgCl₂ (20 mM final), and different HpFT enzymes (HpFT-4HR, HpFT-4HR(M4), HpFT-4HR(M7), HpFT-6HR, HpFT-3HR, HpFT-2HR, HpFT-1HR or HpFT-0HR) (0.5 mg/mL final) in sequence, and the resulting mixed solution was allowed to react at 30 °C for 1 h. Three parallels were set up for each group. After the reaction time point was reached, to the reaction solution was added 10 mM LacNA, and the reaction was terminated by a desalting column (Zeba^{™} Spin Desalting Columns/2 ml/40K, Thermo Fisher Scientific), and finally mass spectrometry was carried out and % of conversion was calculated. % of conversion = (MAR0 peak intensity*0+MAR1peak intensity*1+MAR2 peak intensity*2)/2* (MAR0 peak intensity+MAR1 peak intensity+MAR2 peak intensity)* 100 %. The results are shown in Figure 2. It can be seen that HpFT-3HR, HpFT-2HR, and HpFT-1HR all have significantly improved catalytic activity, as compared with HpFT-4HR. HpFT-0HR has significantly decreased activity, as compared with HpFT-4HR. HpFT-4HR(M4), HpFT-4HR(M7) and HpFT-6HR have no obvious activity change relative to HpFT-4HR.

### Example 19 Comparison of the reaction efficiency of different HpFTs in transferring GDP-FAmP₄Biotin to Trastuzumab-(Fucα1,6)(GalNAzβ1,4)GlcNAc

To 40 mM Tris-HCl (pH 7.5) buffer were added Trastuzumab-(Fucα1,6)(GalNAzβ1,4)GlcNAc (2 mg/mL final), GDP-FAmP₄Biotin (1 mM final), MgCl₂ (20 mM final), and different HpFT enzymes (HpFT-4HR, HpFT-4HR(M4), HpFT-4HR(M7), HpFT-6HR, HpFT-3HR, HpFT-2HR, HpFT-1HR or HpFT-0HR) (0.5 mg/mL final) in sequence, and the resulting mixed solution was allowed to react at 30 °C for 3 h. Three parallels were set up for each group. After the reaction time point was reached, to the reaction solution was added 10 mM LacNAc, and the reaction was terminated by a desalting column (Zeba^{™} Spin Desalting Columns/2 ml/40K, Thermo Fisher Scientific), and finally mass spectrometry was carried out and % of conversion was calculated. % of conversion was calculated according to Example 18. The results are shown in Figure 3. It can be seen that HpFT-3HR, HpFT-2HR, and HpFT-1HR all have significantly improved catalytic activity, as compared with HpFT-4HR. Among them, HpFT-2HR and HpFT-1HR have relatively high catalytic activity. HpFT-0HR has significantly decreased activity, as compared with HpFT-4HR. HpFT-4HR(M4), HpFT-4HR(M7) and HpFT-6HR have little activity change relative to HpFT-4HR.

### Example 20 Comparison of the reaction efficiency of different HpFTs in transferring GDP-FAmP₄Biotin to Trastuzumab-(GalNAzβ1,4) GlcNAc

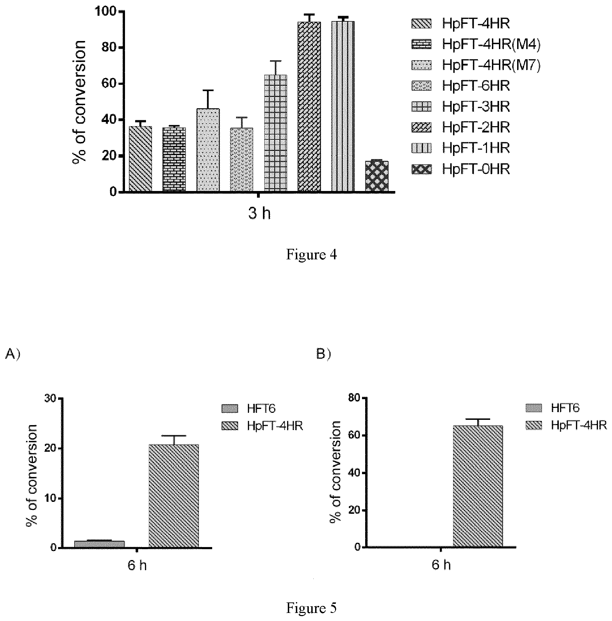

To 40 mM Tris-HCl (pH 7.5) buffer were added Trastuzumab-(GalNAzβ1,4) GlcNAc (2 mg/mL final), GDP-FAmP₄Biotin (1 mM final), MgCl₂ (20 mM final), and different HpFT enzymes (HpFT-4HR, HpFT-4HR(M4), HpFT-4HR(M7), HpFT-6HR, HpFT-3HR, HpFT-2HR, HpFT-1HR or HpFT-0HR) (0.5 mg/mL final) in sequence, and the resulting mixed solution was allowed to react at 30 °C for 3 h. Three parallels were set up for each group. After the reaction time point was reached, to the reaction solution was added 10 mM LacNAc, and the reaction was terminated by a desalting column (Zeba^{™} Spin Desalting Columns/2 ml/40K, Thermo Fisher Scientific), and finally mass spectrometry was carried out and % of conversion was calculated. % of conversion was calculated according to Example 18. The results are shown in Figure 4. It can be seen that HpFT-3HR, HpFT-2HR, and HpFT-1HR all have significantly improved catalytic activity, as compared with HpFT-4HR. Among them, HpFT-2HR and HpFT-1HR have relatively high catalytic activity. HpFT-0HR has significantly decreased activity, as compared with HpFT-4HR. HpFT-4HR(M4), HpFT-4HR(M7) and HpFT-6HR have little activity change relative to HpFT-4HR.

### Example 21 Comparison of the reaction efficiency of HpFT-4HR and HFT6 in transferring GDP-FAmP₄Biotin to Trastuzumab-G₂F and Trastuzumab-(GalNAzβ1,4)GlcNAc

To 40 mM Tris-HCl (pH 7.5) buffer were added Trastuzumab-G₂F orTrastuzumab-(GalNAz(31,4)GlcNAc (2 mg/mL final), GDP-FAmP₄Biotin (1 mM final), MgCl₂ (20 mM final), and HpFT-4HR or HFT6 (0.5 mg/mL final) in sequence, and the resulting mixed solution was allowed to react at 30 °C for 6 h. Three parallels were set up for each group. After the reaction time point was reached, to the reaction solution was added 10 mM LacNAc, and the reaction was terminated by a desalting column (Zeba^{™} Spin Desalting Columns/2 ml/40K, Thermo Fisher Scientific), and finally mass spectrometry was carried out and % of conversion was calculated. For Trastuzumab-G₂F, % of conversion was calculated according to Example 17. For Trastuzumab-(GalNAzβ1,4)GlcNAc, % of conversion was calculated according to Example 18. The results are shown in Figure 5. It can be seen that the activity of *Helicobacter pylori*-derived fucosyltransferase HpFT-4HR is much higher than that of human-derived fucosyltransferase HFT6. On Trastuzumab-G₂F, HFT6 displays very low catalytic activity. On Trastuzumab-(GalNAzβ1,4)GlcNAc, almost no catalytic activity of HFT6 was observed. It should be noted that in the case of preparation of an antibody conjugate using human-derived fucosyltransferase HFT6, it is difficult to obtain a high % of conversion due to its low efficiency and it thus is difficult to obtain a uniform conjugate having a MAR close to the theoretical MAR, thereby limiting its use in the preparation of antibodies.

### Example 22 Comparison of the reaction efficiency of HpFT-2HR and HpFT-4HR in transferring GDP-FAmP₄Biotin or GDP-FAzP₄Biotin to Trastuzumab-G₂F

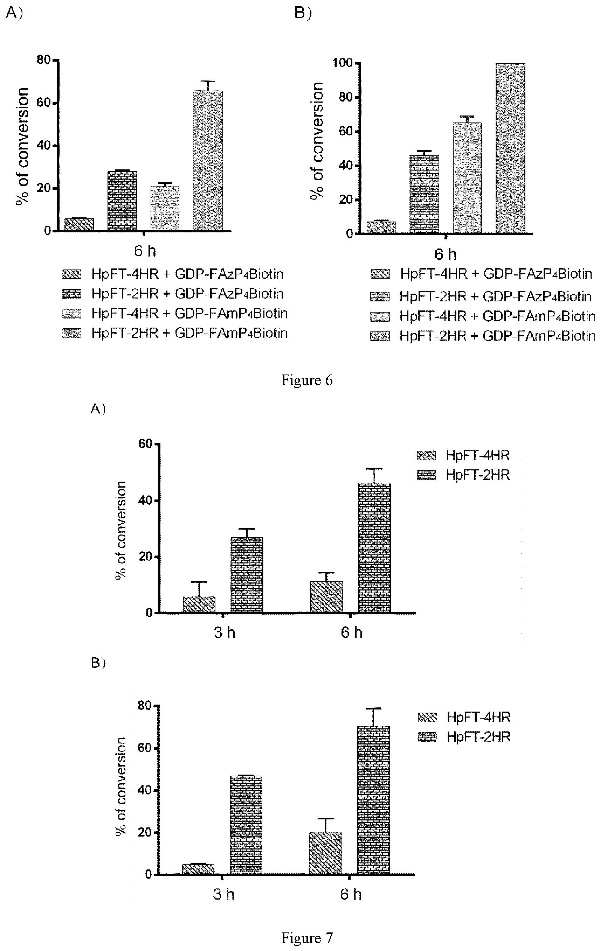

To 40 mM Tris-HCl (pH 7.5) buffer were added Trastuzumab-G₂F (2 mg/mL final), GDP-FAmP₄Biotin or GDP-FAzP₄Biotin (1 mM final), MgCl₂ (20 mM final), and HpFT-2HR or HpFT-4HR (0.5 mg/mL final) in sequence, and the resulting mixed solution was allowed to react at 30 °C for 6 h. Three parallels were set up for each group. After the reaction time point was reached, to the reaction solution was added 10 mM LacNAc, and the reaction was terminated by a desalting column (Zeba^{™} Spin Desalting Columns/2 ml/40K, Thermo Fisher Scientific), and finally mass spectrometry was carried out and % of conversion was calculated. % of conversion was calculated according to Example 17. The results are shown in Figure 6(A). It can be seen that HpFT-2HR has significantly improved activity in catalyzing the transfer of GDP-FAmP₄Biotin and GDP-FAzP₄Biotin, as compared with HpFT-4HR. On the other hand, the activity of HpFT-2HR and HpFT-4HR in catalyzing the transfer of GDP-FAmP₄Biotin is significantly higher than their activity in catalyzing transfer of GDP-FAzP₄Biotin.

### Example 23 Comparison of the reaction efficiency of HpFT-2HR and HpFT-4HR in transferring GDP-FAmP₄Biotin or GDP-FAzP₄Biotin to Trastuzumab-(GalNAzβ1,4)GlcNAc

To 40 mM Tris-HCl (pH 7.5) buffer were added Trastuzumab-(GalNAzβ1,4)GlcNAc (2 mg/mL final), GDP-FAmP₄Biotin or GDP-FAzP₄Biotin (1 mM final), MgCl₂ (20 mM final), and HpFT-2HR or HpFT-4HR (0.5 mg/mL final) in sequence, and the resulting mixed solution was allowed to react at 30 °C for 6 h. Three parallels were set up for each group. After the reaction time point was reached, to the reaction solution was added 10 mM LacNAc, and the reaction was terminated by a desalting column (Zeba^{™} Spin Desalting Columns/2 ml/40K, Thermo Fisher Scientific), and finally mass spectrometry was carried out and % of conversion was calculated. % of conversion was calculated according to Example 18. The results are shown in Figure 6(B). It can be seen that HpFT-2HR has significantly improved activity in catalyzing the transfer of GDP-FAmP₄Biotin and GDP-FAzP₄Biotin, as compared with HpFT-4HR. On the other hand, the activity of HpFT-2HR and HpFT-4HR in catalyzing the transfer of GDP-FAmP₄Biotin is significantly higher than their activity in catalyzing transfer of GDP-FAzP₄Biotin.

### Example 24 Comparison of the reaction efficiency of HpFT-2HR and HpFT-4HR in transferring GDP-FAmSucMMAE to Trastuzumab-G₂F or Durvalumab-G₂F

To 40 mM Tris-HCl (pH 7.5) buffer were added Trastuzumab-G₂F or Durvalumab-G₂F (2 mg/mL final), GDP-FAmSucMMAE (1 mM final), MgCl₂ (20 mM final), and HpFT-2HR or HpFT-4HR (0.5 mg/mL final) in sequence, and the resulting mixed solution was allowed to react at 30 °C for 3 h and 6 h. Three parallels were set up for each group. After the reaction time point was reached, to the reaction solution was added 10 mM LacNAc, and the reaction was terminated by a desalting column (Zeba^{™} Spin Desalting Columns/2 ml/40K, Thermo Fisher Scientific), and finally mass spectrometry was carried out and % of conversion was calculated. % of conversion was calculated according to Example 17. The results are shown in Figure 7. It can be seen that HpFT-2HR has significantly improved activity in catalyzing the transfer of GDP-FAmSucMMAE containing cytotoxins to Trastuzumab-G₂F (Figure 7A) or Durvalumab-G₂F (Figure 7B), as compared with HpFT-4HR.

### Example 25 Comparison of the reaction efficiency of HpFT-2HR and HpFT-4HR in transferring GDP-FAmSucMMAE to Trastuzumab-(GalNAzβ1,4)GlcNAc or Durvalumab-(GalNAzβ1,4)GlcNAc

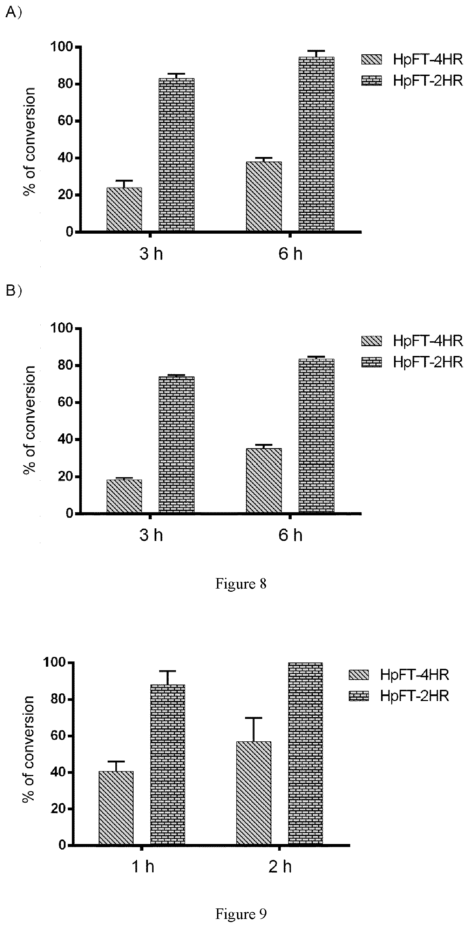

To 40 mM Tris-HCl (pH 7.5) buffer were added Trastuzumab-(GalNAzβ1,4)GlcNAc or Durvalumab-(GalNAzβ1,4)GlcNAc (2 mg/mL final), GDP-FAmSucMMAE (1 mM final), MgCl₂ (20 mM final), and HpFT-2HR or HpFT-4HR (0.5 mg/mL final) in sequence, and the resulting mixed solution was allowed to react at 30 °C for 3 h and 6 h. Three parallels were set up for each group. After the reaction time point was reached, to the reaction solution was added 10 mM LacNAc, and the reaction was terminated by a desalting column (Zeba^{™} Spin Desalting Columns/2 ml/40K, Thermo Fisher Scientific), and finally mass spectrometry was carried out and % of conversion was calculated. % of conversion was calculated according to Example 18. The results are shown in Figure 8. It can be seen that HpFT-2HR has significantly improved activity in catalyzing the transfer of GDP-FAmSucMMAE containing cytotoxins to Trastuzumab-(GalNAzβ1,4)GlcNAc (Figure 8A) or Durvalumab-(GalNAzβ1,4)GlcNAc (Figure 8B), as compared with HpFT-4HR.

### Example 26 Comparison of the reaction efficiency of HpFT-2HR and HpFT-4HR in transferring GDP-FAmSucMMAE to Trastuzumab-(Galβ1,4)GlcNAc

To 40 mM Tris-HCl (pH 7.5) buffer were added Trastuzumab-(Galβ1,4)GlcNAc (2 mg/mL final), GDP-FAmSucMMAE (1 mM final), MgCl₂ (20 mM final), and HpFT-2HR or HpFT-4HR (0.5 mg/mL final) in sequence, and the resulting mixed solution was allowed to react at 30 °C for 1 h and 2 h. Three parallels were set up for each group. After the reaction time point was reached, to the reaction solution was added 10 mM LacNAc, and the reaction was terminated by a desalting column (Zeba^{™} Spin Desalting Columns/2 ml/40K, Thermo Fisher Scientific), and finally mass spectrometry was carried out and % of conversion was calculated. % of conversion was calculated according to Example 18. The results are shown in Figure 9. It can be seen that HpFT-2HR has significantly improved activity in catalyzing the transfer of GDP-FAmSucMMAE containing cytotoxins to Trastuzumab-(Galβ1,4)GlcNAc, as compared with HpFT-4HR.

### Example 27 Preparation of Trastuzumab-G₂F-Fuc* antibody conjugate using HpFT-2HR

To 50 mM Tris-HCl (pH 7.0) buffer were added Trastuzumab-G₂F (3 mg/mL final), GDP-Fuc* (GDP-FAmAz, GDP-FAmP₄Tz, GDP-FAmSucMMAE or GDP-FAmAzP₄DXd) (1.5 mM final), MgCl₂ (20 mM final), and HpFT-2HR (0.5 mg/mL final) in sequence, and the resulting mixed solution was allowed to react at 30 °C for 16-48 h. Then, the reaction solution was purified with protein A resin to give the modified antibody of interest. Mass spectral analysis showed the major peaks of Trastuzumab-G₂F-FAmAz (149687 Da, MAR 4), Trastuzumab-G₂F-FAmP₄Tz (151032 Da, MAR 4), Trastuzumab-G2F-FAmSucMMAE (154178 Da, MAR 4), and Trastuzumab-G₂F-FAmAzP₄DXd (154017 Da, MAR 4), respectively. For Trastuzumab-G₂F-FAmSucMMAE, in addition to the major peak with a molecular weight of 154178 Da, one more minor peak with a molecular weight of 153414 Da appeared due to the fragmentation of vc-PAB moiety during MS spectrometry. Similar fragmentation peaks also appeared for following antibody conjugates containing the vc-PAB moiety.

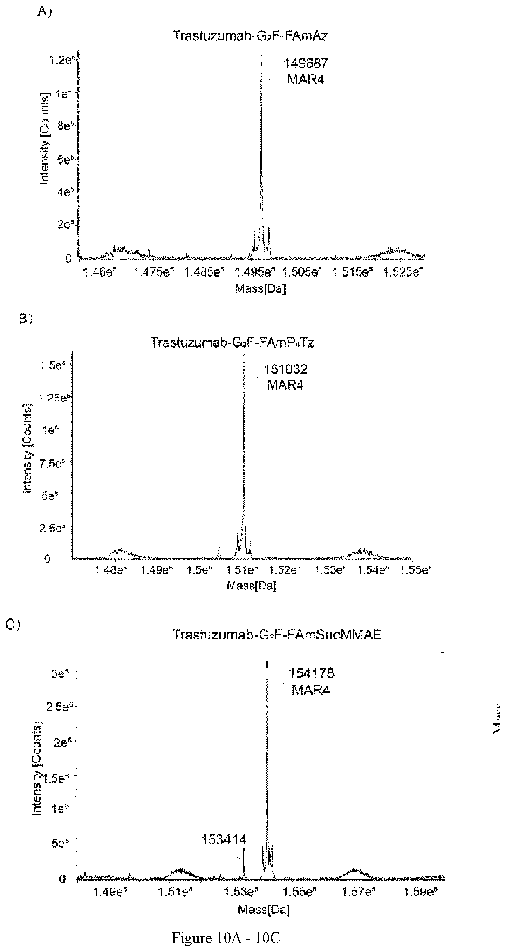

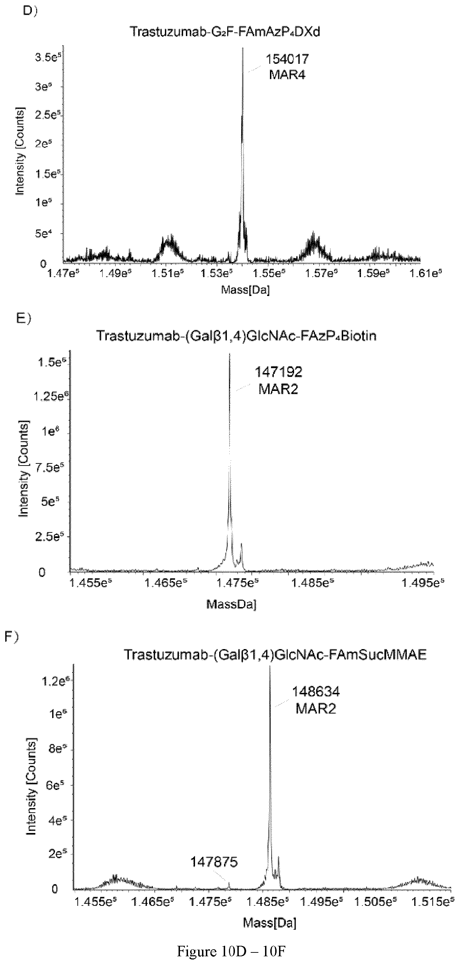

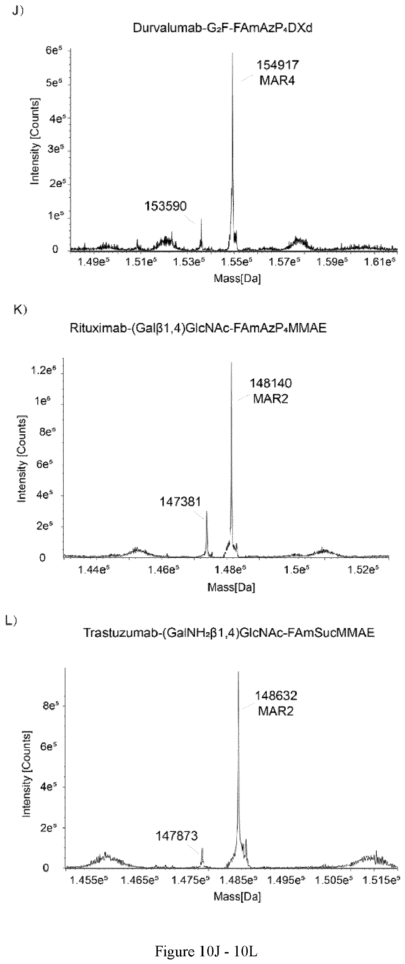

The results are shown in Figures 10A-10D, indicating that the obtained antibody conjugates have good homogeneity and their MARs are all between 3.6 and 4.0.

### Example 28 Preparation of Durvalumab-G₂F-Fuc* antibody conjugate using HpFT-2HR

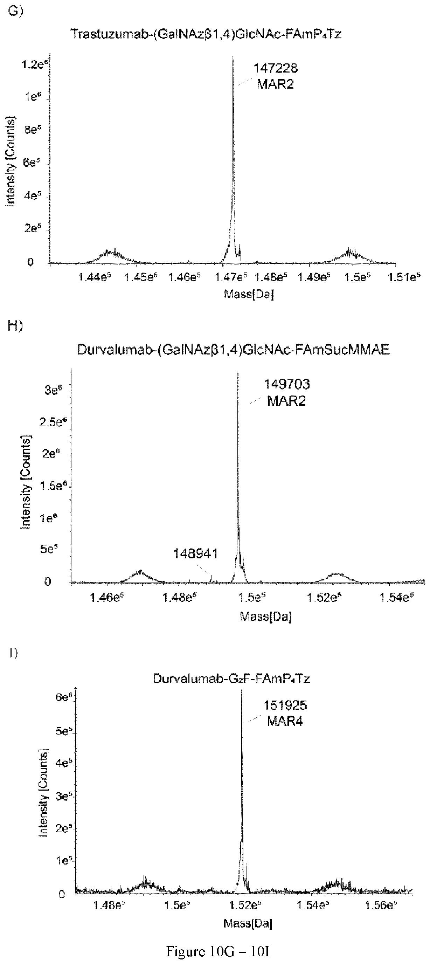

To 50 mM Tris-HCl (pH 7.0) buffer were added Durvalumab-G₂F (3 mg/mL final), GDP-Fuc* (GDP-FAmP₄Tz or GDP-FAmAzP₄DXd) (1.5 mM final), MgCl₂ (20 mM final), and HpFT-2HR (0.5 mg/mL final) in sequence, and the resulting mixed solution was allowed to react at 30 °C for 24-48 h. Then, the reaction solution was purified with protein A resin to give the modified antibody of interest. Mass spectral analysis showed the major peaks of Durvalumab-G₂F-FAmP₄Tz (151925 Da, MAR 4) and Durvalumab-G₂F-FAmAzP₄DXd (154917 Da, MAR 4), respectively. For Durvalumab-G₂F-FAmAzP₄DXd, in addition to the major peak with a molecular weight of 154917 Da, there was also one more minor peak with a molecular weight of 153590 Da, which was a MAR3 product given by linking a trimolecular FAmAzP₄DXd to an antibody molecule. The results are shown in Figures 10I-10J, indicating that the obtained antibody conjugates have good homogeneity and their MARs are all between 3.6 and 4.0.

### Example 29 Preparation of Trastuzumab-(Galβ1,4)GlcNAc-Fuc* using HpFT-2HR

To 50 mM Tris-HCl (pH 7.0) buffer were added Trastuzumab-(Galβ1,4)GlcNAc (3 mg/mL final), GDP-Fuc* (GDP-FAmSucMMAE or GDP-FAzP₄Biotin) (1.5 mM final), MgCl₂ (20 mM final), and HpFT-2HR (0.5 mg/mL final) in sequence, and the resulting mixed solution was allowed to react at 30 °C for 2-16 h. Then, the reaction solution was purified with protein A resin to give the modified antibody of interest. Mass spectral analysis showed the major peaks of Trastuzumab-(Galβ1,4)GlcNAc-FAmSucMMAE (148634 Da, MAR 2) and Trastuzumab-(Galβ1,4)GlcNAc-FAzP₄Biotin (147192 Da, MAR 2), respectively. The results are shown in Figures 10E-10F, indicating that the obtained antibody conjugates have good homogeneity and their MARs are between 1.8 and 2.0.

### Example 30 Preparation of Trastuzumab-(GalNAzβ1,4)GlcNAc-Fuc* using HpFT-2HR

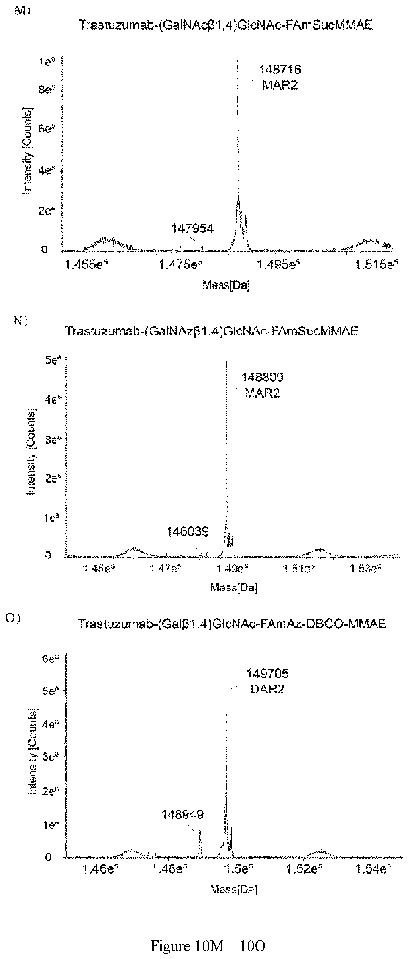

To 50 mM Tris-HCl (pH 7.0) buffer were added Trastuzutnab-(GalNAzβ1,4)GlcNAc (3 mg/mL final), GDP-Fuc* (GDP-FAmP₄Tz or GDP-FAmSucMMAE) (1.5 mM final), MgCl₂ (20 mM final), and HpFT-2HR (0.5 mg/mL final) in sequence, and the resulting mixed solution was allowed to react at 30 °C for 12-16 h. Then, the reaction solution was purified with protein A resin to give the modified antibody of interest. Mass spectral analysis showed the major peaks of Trastuzumab-(GalNAzβ1,4)GlcNAc-FAmP₄Tz (147228 Da, MAR 2) and Trastuzumab-(GalNAzβ1,4)GlcNAc-FAmSucMMAE (148800 Da, MAR 2), respectively. The results are shown in Figures 10G and 10N, indicating that the obtained antibody conjugates have good homogeneity and their MARs are between 1.8 and 2.0.

### Example 31 Preparation of Durvalumab-(GalNAzβ1,4)GlcNAc-FAmSucMMAE using HpFT-2HR

To 50 mM Tris-HCl (pH 7.0) buffer were added Durvalumab-(GalNAzβ1,4)GlcNAc (3 mg/mL final), GDP-FAmSucMMAE (1.5 mM final), MgCl₂ (20 mM final), and HpFT-2HR (0.5 mg/mL final) in sequence, and the resulting mixed solution was allowed to react at 30 °C for 16 h. Then, the reaction solution was purified with protein A resin to give the modified antibody of interest. Mass spectral analysis showed the major peak of Durvalumab-(GalNAzβ1,4)GlcNAc-FAmSucMMAE (149703 Da, MAR 2). The results are shown in Figure 10H, indicating that the obtained antibody conjugates have good homogeneity and their MARs are between 1.8 and 2.0.

### Example 32 "One-pot" preparation of Rituximab-(Galβ1,4)GlcNAc-FAmAzP₄MMAE using HpFT-2HR

First, to 50 mM Tris-HCl (pH 7.0) buffer were added Rituximab (3 mg/mL final), EndoS (0.05 mg/mL final) and Alfc (1 mg/mL final) in sequence, and the resulting mixed solution was allowed to react at 37 °C for 24 h. Then, to the reaction solution were added UDP-galactose (5 mM final), GDP-FAmAzP₄MMAE (1.5 mM final), MnCl₂ (5 mM final), MgCl₂ (20 mM final), BGalT1(Y289L) (0.5 mg/mL final), and HpFT-2HR (0.5 mg/mL final) in sequence, and the resulting mixed solution was allowed to further react at 30 °C for 16 h. Then, the reaction solution was purified with protein A resin to give the modified antibody of interest. Mass spectral analysis showed the major peak of Rituximab-(Galβ1,4)GlcNAc-FAmAzP₄MMAE (148140 Da, MAR2). The results are shown in Figure 10K, indicating that the obtained antibody conjugates have good homogeneity and their MARs are between 1.8 and 2.0. The light chain of Rituximab has an amino acid sequence as set forth in SEQ ID NO: 21, and the heavy chain has an amino acid sequence as set forth in SEQ ID NO: 22.

### Example 33 Preparation of Trastuzumab-(GalNH₂β1,4)GlcNAc-FAmSucMMAE using HpFT-2HR

First, to 50 mM Tris-HCl (pH 7.0) buffer were added Trastuzumab (5 mg/mL final), EndoS (0.05 mg/mL final) and Alfc (1.5 mg/mL final) in sequence, and the resulting mixed solution was allowed to react at 37 °C for 24 h. Then, to the reaction solution were added UDP-GalNH₂ (uridine diphosphate-2-galactosamine) (5 mM final), MnCl₂ (5 mM final), and BGalT1(Y289L) (0.5 mg/mL final) in sequence, and the resulting mixed solution was allowed to further react at 30 °C for 48 h. Then, the reaction solution was purified with protein A resin to give the modified antibody of interest Trastuzumab-(GalNH₂β1,4)GlcNAc. Then, to 50 mM Tris-HCl (pH 7.0) buffer were added Trastuzumab-(GalNH₂β1,4)GlcNAc (3 mg/mL final), GDP-FAmSucMMAE (1.5 mM final), MgCl₂ (20 mM final), and HpFT-2HR (0.5 mg/mL final) in sequence, and the resulting mixed solution was allowed to react at 30 °C for 16 h. Then, the reaction solution was purified with protein A resin to give the modified antibody of interest. Mass spectral analysis showed the major peak of Trastuzumab-(GalNH₂β1,4)GlcNAc-FAmSucMMAE (148632 Da, MAR2). The results are shown in Figure 10L, indicating that the obtained antibody conjugates have good homogeneity and their MARs are between 1.8 and 2.0.

### Example 34 "One-pot" preparation of Trastuzumab-(GalNAcβ1,4)GlcNAc-FAmSucMMAE using HpFT-2HR

First, to 50 mM Tris-HCl (pH 7.0) buffer were added Trastuzumab (3 mg/mL final), EndoS (0.05 mg/mL final) and Alfc (1 mg/mL final) in sequence, and the resulting mixed solution was allowed to react at 37 °C for 24 h. Then, to the reaction solution were added UDP-GalNAc (uridine diphosphate-N-acetylgalactosamine) (5 mM final), GDP-FAmSucMMAE (1.5 mM final), MnCl₂ (5 mM final), MgCl₂ (20 mM final), BGalT1(Y289L) (0.5 mg/mL final), and HpFT-2HR (0.5 mg/mL final) in sequence, and the resulting mixed solution was allowed to further react at 30 °C for 16 h. Then, the reaction solution was purified with protein A resin to give the modified antibody of interest. Mass spectral analysis showed the major peak of Trastuzutnab-(GaLNAcβ1,4)GlcNAc-FAmSucMMAE (148716 Da, MAR2). The results are shown in Figure 10M, indicating that the obtained antibody conjugates have good homogeneity and their MARs are between 1.8 and 2.0.

### Example 35 Preparation of Trastuzumab-(Galβ1,4)GlcNAc-FAmSucMMAE using HpFT-1HR

To 50 mM Tris-HCl (pH 7.0) buffer were added Trastuzumab-(Galβ1,4)GlcNAc (3 mg/mL final), GDP-FAmSucMMAE (1.5 mM final), MgCl₂ (20 mM final), and HpFT-1HR (0.5 mg/mL final) in sequence, and the resulting mixed solution was allowed to react at 30 °C for 6 h. Then, the reaction solution was purified with protein A resin to give the modified antibody of interest. Mass spectral analysis showed the major peak of Trastuzumab-(Galβ1,4)GlcNAc-FAmSucMMAE (148634 Da, MAR 2). The obtained antibody conjugates have good homogeneity and their MARs are between 1.8 and 2.0.

### Example 36 Preparation of Trastuzumab-(Galβ1,4)GlcNAc-FAmSucMMAE using HpFT-3HR

To 50 mM Tris-HCl (pH 7.0) buffer were added Trastuzumab-(Galβ1,4)GlcNAc (3 mg/mL final), GDP-FAmSucMMAE (1.5 mM final), MgCl₂ (20 mM final), and HpFT-3HR (0.5 mg/mL final) in sequence, and the resulting mixed solution was allowed to react at 30 °C for 6 h. Then, the reaction solution was purified with protein A resin to give the modified antibody of interest. Mass spectral analysis showed the major peak of Trastuzumab-(Galβ1,4)GlcNAc-FAmSucMMAE (148634 Da, MAR 2). The obtained antibody conjugates have good homogeneity and their MARs are between 1.8 and 2.0.

### Example 37 Preparation of Trastuzumab-G₂F-FAmP₄Tz-TCO-MMAE using Trastuzumab-G₂F-FAmP₄Tz

To 1×PBS (pH 7.0) buffer were added Trastuzumab-G₂F-FAmP₄Tz (1 mg/mL final) and TCO-PEG₄-vc-PAB-MMAE (0.5 mM final) (Levena Biopharma) in sequence, and the resulting mixed solution was allowed to react at r.t. for 4 h. Then, the reaction solution was purified with protein A resin to give the modified antibody of interest. Mass spectral analysis showed the major peak of Trastuzumab-G₂F-FAmP₄Tz-TCO-MMAE (157007 Da, DAR 4). The obtained antibody conjugates have good homogeneity and their DARs are between 3.6 and 4.0.

### Example 38 Preparation of Trastuzumab-(Galβ1,4)GlcNAc-FAmAz-DBCO-MMAE using Trastuzumab-(Galβ1,4)GlcNAc-FAmAz

First, to 50 mM Tris-HCl (pH 7.0) buffer were added Trastuzumab-(Galβ1,4)GlcNAc (3 mg/mL final), GDP-FAmAz (1 mM final), MgCl₂ (5 mM final), and HpFT-2HR (0.5 mg/mL final) in sequence, and the resulting mixed solution was allowed to react at 30 °C for 6 h. Then, the reaction solution was purified with protein A resin to give the modified antibody of interest Trastuzumab-(Galβ1,4)GlcNAc-FAmAz.

To 1×PBS (pH 7.0) buffer were added Trastuzumab-(Galβ1,4)GlcNAc-FAmAz (1 mg/mL final) and DBCO-PEG₄-vc-PAB-MMAE (0.5 mM final) (Levena Biopharma) in sequence, and the resulting mixed solution was allowed to react at r.t. for 16 h. Then, the reaction solution was purified with protein A resin to give the modified antibody of interest. Mass spectral analysis showed the major peak of Trastuzumab-(Galβ1,4)GlcNAc-FAmAz-DBCO-MMAE (149705 Da, DAR 2). The results are shown in Figure 10O, indicating that the obtained antibody conjugates have good homogeneity and their DARs are between 1.8 and 2.0.

### Example 39 In vitro efficacy evaluation of Trastuzumab-G₂F-FAmSucMMAE and Trastuzumab-G₂F-FAmP₄Tz-TCO-MMAE on SK-Br-3 and MDA-MB-231 cells

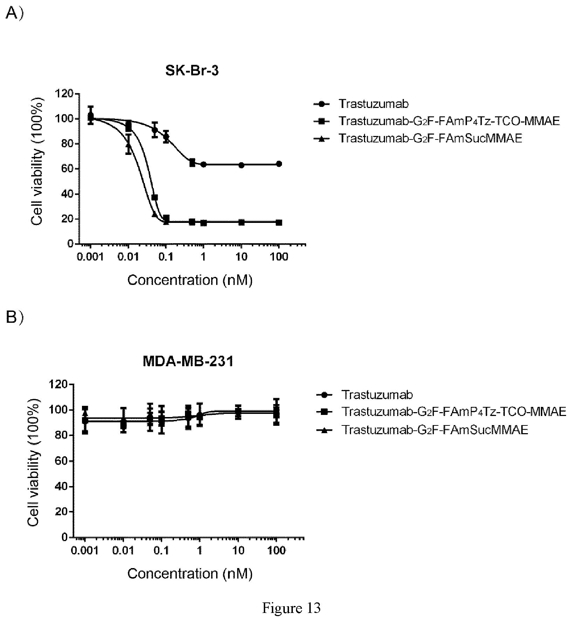

SK-Br-3 (Her2+) cells were cultured in RPMI 1640 medium (Gibco) supplemented with 10% FBS (Gibco). MDA-MB-231 (Her2-) cells were cultured in DMEM (Gibco) supplemented with 10% FBS (Gibco). The two types of cells in good culture condition were respectively plated in 96-well plates with 5000 cells per well and then incubated for 24 h in a constant-temperature cell incubator at 37 °C and 5% CO2. After the culture, the culture medium was removed from the 96-well plates, and 200 µL fresh culture medium with drug (Trastuzumab, Trastuzumab-G₂F-FAmSucMMAE, or Trastuzumab-G₂F-FAmP₄Tz-TCO-MMAE) (final concentrations were 100 nM, 10 nM, 1 nM, 0.05 nM, 0.1 nM, 0.05 nM, 0.01 nM, 0.001 nM and 0 nM respectively) was then added to the plates. After the sample loading, the cells in the 96-well plates were further incubated for 72 h in the constant-temperature cell incubator. After the incubation, the cells were subjected to a CellTiter-Glo^{®} Luminescent Cell Viability Assay (Promega) to measure the killing effect of the drug to be tested on SK-Br-3 and MDA-MB-231. The results are shown in Figure 13. The results show that Trastuzumab-G₂F-FAmSucMMAE and Trastuzumab-G₂F-FAmP₄Tz-TCO-MMAE have a strong killing effect on SK-Br-3 (Her2+) cells, but have no obvious killing effect on MDA-MB-231 (Her2-) cells.

## Claims

1. An isolated polypeptide, comprising a catalytically active domain and one or more heptapeptide repeat fragments, said catalytically active domain comprising an amino acid sequence as set forth in SEQ ID NO: 1, said heptapeptide repeat fragment comprising an amino acid sequence as set forth in SEQ ID NO: 2, and in the isolated polypeptide, the number of said heptapeptide repeat fragments being not more than 3.

2. The isolated polypeptide of claim 1, wherein in the polypeptide, said catalytically active domain is located at an N-terminus of said heptapeptide repeat fragment.

3. The isolated polypeptide of any one of claims 1-2, wherein the number of said heptapeptide repeat fragments is 1.

4. The isolated polypeptide of claim 3, comprising an amino acid sequence as set forth in SEQ ID NO: 3.

5. The isolated polypeptide of any one of claims 1-2, wherein the number of said heptapeptide repeat fragments is 2, and two said heptapeptide repeat fragments are directly linked to each other.

6. The isolated polypeptide of claim 5, comprising an amino acid sequence as set forth in SEQ ID NO: 4.

7. The isolated polypeptide of any one of claims 1-2, wherein the number of said heptapeptide repeat fragments is 3, and three said heptapeptide repeat fragments are directly linked in sequence.

8. The isolated polypeptide of claim 7, comprising an amino acid sequence as set forth in SEQ ID NO: 5.

9. The isolated polypeptide of any one of claims 1-8, further comprising a tag sequence.

10. The isolated polypeptide of claim 9, wherein said tag sequence is an affinity purification tag.

11. The isolated polypeptide of any one of claims 1-10, comprising an amino acid sequence as set forth in any one of SEQ ID NOs: 3-5 and 8-10.

12. One or more isolated nucleic acid molecules, encoding the isolated polypeptide of any one of claims 1-11.

13. A vector comprising the nucleic acid sequence of the nucleic acid molecule of claim 12.

14. A cell comprising and/or expressing the isolated polypeptide of any one of claims 1-11, the nucleic acid molecule of claim 12, and/or the vector of claim 13.

15. A catalytic preparation comprising the isolated polypeptide of any one of claims 1-11.

16. A composition comprising the isolated polypeptide of any one of claims 1-11.

17. A method for transferring a fucose and/or a fucose derivative, comprising: using the isolated polypeptide of any one of claims 1-11, the nucleic acid molecule of claim 12, the vector of any one of claim 13, and/or the cell of claim 14.

18. A use of the isolated polypeptide of any one of claims 1-11 in the preparation of a protein conjugate.

19. A method for preparing a protein conjugate, comprising: using the isolated polypeptide of any one of claims 1-11, the nucleic acid molecule of claim 12, the vector of claim 13, the cell of claim 14, and/or the catalytic preparation of claim 15.

20. The method of claim 19, comprising:
in the presence of the isolated polypeptide of any one of claims 1-11 and/or the catalytic preparation of claim 15, contacting a donor Q-Fuc* with a protein having a glycan chain comprising a structure of formula (I) to obtain a protein conjugate, said protein conjugate having a glycan chain comprising a structure of formula (II):
-GlcNAc(Fuc)_{b}-GalX, formula (I); 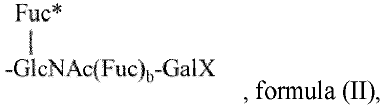
wherein
said GlcNAc is a N-acetylglucosamine;
Fuc is a fucose, b is 0 or 1;
GalX is an optionally substituted galactose;
Fuc* comprises a structure of Fuco-MOI, wherein the structure of Fuco is represented by formula (III): 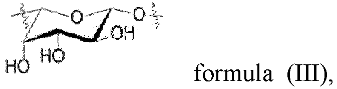 MOI is a molecule of interest comprising an active substance (AM), said MOI is linked to the left terminus of formula (III), and
Q is a ribonucleotide diphosphate.

21. The method of claim 20, wherein said protein comprises a Fc domain and/or an antigen-binding moiety, said GalX and said GlcNAc are linked via a β-1,4 glycosidic linkage, said Fuc and said GlcNAc are linked via an α-1,6 glycosidic linkage, and said Fuc* and said GlcNAc are linked via an α-1,3 glycosidic linkage.

22. The method of any one of claims 20-21 wherein said GalX is a galactose.

23. The method of any one of claims 20-21, wherein said GalX is a substituted galactose, and one or more hydroxyls at positions C2, C3, C4 and/or C6 in said galactose are substituted.

24. The method of claim 23, wherein said GalX is a substituted galactose, and a hydroxyl at position C2 in said galactose is substituted.

25. The method of any one of claims 20-24, wherein said GalX is a monosaccharide.

26. The method of any one of claims 23-25, wherein said GalX is substituted with a substituent Rg¹, and the Rg¹ is a group selected from the group consisting of: hydrogen, halogen, -NH₂, -SH, -N₃, -COOH, -CN, C₁-C₂₄ alkyl group, C₃-C₂₄ cycloalkyl group, C₂-C₂₄ alkenyl group, C₅-C₂₄ cycloalkenyl group, C₂-C₂₄ alkynyl group, C₇-C₂₄ cycloalkynyl group, C₂-C₂₄ (hetero)aryl group, C₃-C₂₄ alkyl(hetero)aryl group, and C₃-C₂₄ (hetero)arylalkyl group;
wherein said alkyl group, cycloalkyl group, alkenyl group, cycloalkenyl group, alkynyl group, cycloalkynyl group, (hetero)aryl group, alkyl(hetero)aryl group, and/or (hetero)arylalkyl group are each independently optionally substituted with one or more substituents Rs¹, and/or each independently optionally spaced by one or more substituents Rs²;
wherein each of said Rs1(s) is independently a group selected from the group consisting of: halogen, -OH, -NH₂, -SH, -N₃, -COOH, and -CN;
each of said Rs² (s) is independently a group selected from the group consisting of: -O-, -S-, 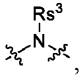 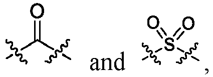 wherein said Rs³ is a group selected from the group consisting of: hydrogen, C₁-C₂₄ alkyl group, C₂-C₂₄ alkenyl group, C₂-C₂₄ alkynyl group, and C₃-C₂₄ cycloalkyl group, wherein said C₁-C₂₄ alkyl group, C₂-C₂₄ alkenyl group, C₂-C₂₄ alkynyl group, and C₃-C₂₄ cycloalkyl group are optionally substituted.

27. The method of any one of claims 23-25, wherein said GalX is substituted with a substituent 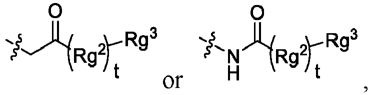 wherein:
t is 0 or 1;
the Rg² is a group selected from the group consisting of: C₁-C₂₄ alkylene group, C₃-C₂₄ cycloalkylene group, C₂-C₂₄ alkenylene group, C₅-C₂₄ cycloalkenylene group, C₂-C₂₄ alkynylene group, C₇-C₂₄ cycloalkynylene group, C₂-C₂₄ (hetero)arylene group, C₃-C₂₄ alkyl(hetero)arylene group, and C₃-C₂₄ (hetero)arylalkylene group, wherein said alkylene group, cycloalkylene group, alkenylene group, cycloalkenylene group, alkynylene group, cycloalkynylene group, (hetero)arylene group, alkyl(hetero)arylene group and/or (hetero)arylalkylene group are each independently optionally substituted with one or more substituents Rs¹, and/or each independently optionally spaced by one or more substituents Rs²,
said Rg³ is a group selected from the group consisting of: hydrogen, halogen, -OH, -NH₂, -SH, -N₃, - COOH, -CN, C₁-C₂₄ alkyl group, C₃-C₂₄ cycloalkyl group, C₂-C₂₄ alkenyl group, C₅-C₂₄ cycloalkenyl group, C₂-C₂₄ alkynyl group, C₇-C₂₄ cycloalkynyl group, and C₂-C₂₄ (hetero)aryl group; wherein said alkyl group, cycloalkyl group, alkenyl group, cycloalkenyl group, alkynyl group, cycloalkynyl group, and/or (hetero)aryl group are each independently optionally substituted with one or more Rs¹(s),
wherein
each of said Rs²(s) is independently selected from the group consisting of: -O-, -S-, 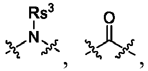 and 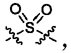 said Rs³ is a group selected from the group consisting of: hydrogen, C₁-C₂₄ alkyl group, C₂-C₂₄ alkenyl group, C₂-C₂₄ alkynyl group, and C₃-C₂₄ cycloalkyl group, wherein said C₁-C₂₄ alkyl group, C₂-C₂₄ alkenyl group, C₂-C₂₄ alkynyl group, and C₃-C₂₄ cycloalkyl group are optionally substituted, and
each of said Rs1(s) is independently a group selected from the group consisting of: halogen, -OH, - NH₂, -SH, -N₃, -COOH, and -CN.

28. The method of any one of claims 20-27, wherein the GlcNAc in said formula (I) is directly linked to an asparagine residue in said protein.

29. The method of any one of claims 20-27, wherein the glycan chain of said protein comprises a mannose, and the GlcNAc in said formula (I) is directly linked to the mannose in said glycan chain.

30. The method of any one of claims 20-29, wherein said protein comprises a Fc domain, and said glycan chain is linked to an asparagine residue of said Fc domain.

31. The method of any one of claims 20-30, wherein said protein comprises a Fc domain, and said glycan chain is linked to an asparagine residue of a CH₂ domain in said Fc domain.

32. The method of any one of claims 20-31, wherein said protein comprise a Fc domain, and said glycan chain is linked to position N297 of said Fc domain, wherein said position is determined according to EU index numbering in Kabat.

33. The method of any one of claims 20-32, wherein said protein is a Fc domain-containing antibody and/or Fc fusion protein.

34. The method of any one of claims 20-33, wherein said protein is a monoclonal antibody.

35. The method of any one of claims 20-34, wherein said protein is an IgG antibody.

36. The method of any one of claims 20-35, wherein said Q is a guanosine diphosphate (GDP), a uridine diphosphate (UDP), and/or a cytidine diphosphate (CDP).

37. The method of any one of claims 20-36, wherein said Q is a guanosine diphosphate (GDP).

38. The method of any one of claims 20-37, wherein said active substance AM comprises a biologically active substance, a pharmaceutically active substance, a chemically reactive substance, and/or an enzymatically reactive substance.

39. The method of any one of claims 20-38, wherein said active substance AM is a chemically reactive substance and/or an enzymatically reactive substance.

40. The method of any one of claims 20-39, wherein said active substance AM is a functional group X₁, and the X₁ comprises a functional group capable of participating in a bioorthogonal ligation reaction.

41. The method of claim 40, wherein said functional group capable of participating in a bioorthogonal ligation reaction is selected from one or more of the group consisting of: azido, terminal alkynyl, cycloalkynyl, tetrazinyl, 1,2,4-triazinyl, terminal alkenyl, cycloalkenyl, keto, aldehyde, hydroxylamino, mercapto, maleimido and their functional derivatives.

42. The method of any one of claims 40-41, wherein said functional group capable of participating in a bioorthogonal ligation reaction is selected from one or more of the group consisting of: 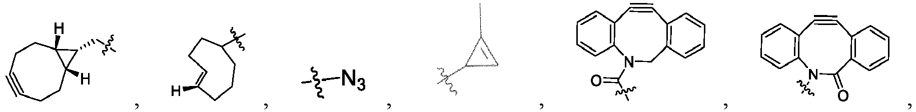 and

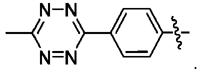

43. The method of any one of claims 20-38, wherein said active substance AM is a biologically active substance and/or a pharmaceutically active substance P1.

44. The method of claim 43, wherein said active substance AM is a pharmaceutically active substance P1.

45. The method of any one of claims 43-44, wherein said P₁ comprises one or more substances selected from the group consisting of: a cytotoxin, an agonist, an antagonist, an antiviral agent, an antibacterial agent, a radioisotope, a radionuclide, a metal chelator, an oligonucleotide, and a polypeptide.

46. The method of any one of claims 43-45, wherein said P₁ comprises a cytotoxin.

47. The method of any one of claims 43-46, wherein said P₁ comprises a cytotoxin and said cytotoxin is selected from one or more of the group consisting of a DNA damaging agent, a topoisomerase inhibitor, an RNA polymerase inhibitor, and a tubulin inhibitor.

48. The method of any one of claims 43-47, wherein said P₁ may comprise a cytotoxin, and said cytotoxin is selected from one or more of the group consisting of: pyrrolobenzodiazepine and a derivative thereof, auristatin and a derivative thereof, maytansinoids and a derivative thereof, duocarmycin and a derivative thereof, tubulysin and a derivative thereof, enediyene and a derivative thereof, doxorubicin and a derivative thereof, pyrrole-based kinesin spindle protein inhibitor and a derivative thereof, calicheamicin and a derivative thereof, amanitin and a derivative thereof, and camptothecin and a derivative thereof.

49. The method of any one of claims 43-48, wherein said P₁ comprises a cytotoxin and said cytotoxin is selected from one or more of the group consisting of: MMAE and DXd.

50. The method of any one of claims 20-49, wherein said MOI further comprises a linker L used for linking said AM to said Fuco.

51. The method of claim 50, wherein said linker L has a structure of J-(Sp)ₙ, wherein n is 0 or 1, J is a connector, Sp is a spacer, and said J is directly linked to said Fuco.

52. The method of claim 51, wherein said J is a structure selected from the group consisting of: 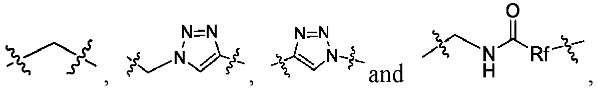 wherein Rf is -CH₂-, -NH- or -O-, wherein the left terminus of said structure is directly linked to said Fuco.

53. The method of any one of claims 51-52, wherein said J is 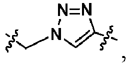 and the left terminus of the structure is directly linked to said Fuco.

54. The method of any one of claims 51-52, wherein said J is 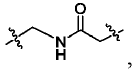 and the left terminus of the structure is directly linked to said Fuco.

55. The method of any one of claims 51-54, wherein said spacer Sp comprises a cleavable moiety.

56. The method of claim 55, wherein said cleavable moiety is selected from one or more of the group consisting of: an acid-labile cleavable moiety, a redox-active cleavable moiety, a photoactive cleavable moiety, and a proteolytically cleavable moiety.

57. The method of any one of claims 55-56, wherein said cleavable moiety is selected from one or more of the group consisting of: vc-PAB and GGFG.

58. The method of any one of claims 51-54, wherein said spacer Sp does not comprise a cleavable moiety.

59. The method of any one of claims 20-58, wherein said Q-Fuc* is a Q-Fuco-L-AM.

60. The method of any one of claims 20-59, wherein said Q-Fuc* is a Q-Fuco-J-(Sp)ₙ-AM, wherein n is 0 or 1.

61. The method of any one of claims 20-42 and 50-60, wherein said Q-Fuc* is a Q-Fuco-J-(Sp)ₙ-X₁, wherein n is 0 or 1.

62. The method of any one of claims 20-38 and 43-60, wherein said Q-Fuc* is a Q-Fuco-J-(Sp)ₙ-P₁, wherein n is 0 or 1.

63. The method of any one of claims 51-62, wherein n is 1.

64. The method of any one of claims 51-62, wherein n is 0.

65. The method of any one of claims 20-64, wherein said GalX is selected from the group consisting of:

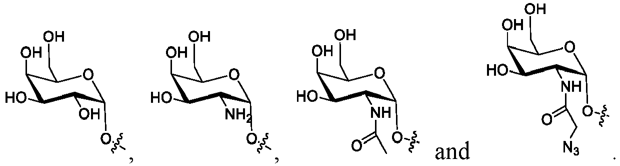

66. The method of any one of claims 20-65, wherein said protein conjugate obtained comprises 1 to 20 structures of formula (II).

67. The method of any one of claims 20-66, wherein said protein conjugate obtained comprises two or four structures of formula (II).

68. The method of any one of claims 20-27 and 29-67, wherein said protein conjugate comprises four structures of formula (II).

69. The method of claim 68, wherein the structure of said protein conjugate obtained is represented by formula (IV): 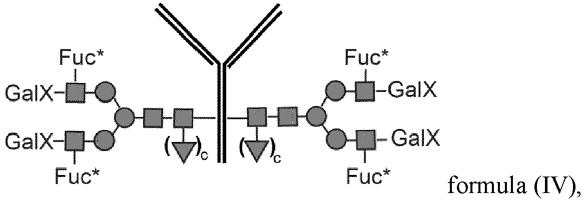 wherein 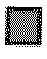 is the N-acetylglucosamine, 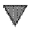 is the fucose, 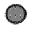 is the mannose, the GalX is said optionally substituted galactose, 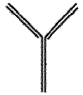 is a Fc domain-containing antibody and/or Fc fusion protein, c is 0 or 1, and said glycan chain is linked to the Fc domain of said Fc domain-containing antibody and/or Fc fusion protein.

70. The method of claim 69, further comprising the following steps:
contacting the Fc domain-containing antibody and/or Fc fusion protein having a G₀(F)_{0,1}, G₁(F)_{0,1} and/or G₂(F)_{0,1} glycoform with a UDP-GaIX in the presence of a suitable catalyst to obtain a protein having a glycan chain comprising a structure of formula (I), the structure of the protein being represented by formula (V): 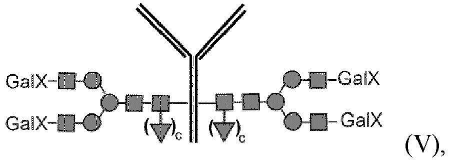
wherein 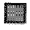 is the N-acetylglucosamine, 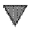 is the fucose, 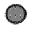 is the mannose, the GalX is the galactose, 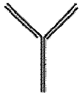 is the Fc domain-containing antibody and/or Fc fusion protein, c is 0 or 1, and said glycan chain is linked to the Fc domain of said Fc domain-containing antibody and/or Fc fusion protein.

71. The method of claim 69, further comprising the following steps:
contacting the Fc domain-containing antibody and/or Fc fusion protein having a G₀(F)_{0,1} glycoform with a UDP-GaIX in the presence of a suitable catalyst to obtain a protein having a glycan chain comprising a structure of formula (I), the structure of the protein being represented by formula (V): 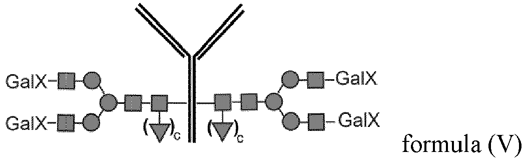
wherein ■ is the N-acetylglucosamine, ▼ is the fucose, • is the mannose, the GalX is said optionally substituted galactose, 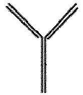 is the Fc domain-containing antibody and/or Fc fusion protein, c is 0 or 1, and said glycan chain is linked to the Fc domain of said Fc domain-containing antibody and/or Fc fusion protein.

72. The method of any one of claims 20-28 and 30-67, wherein said protein conjugate comprises two structures of formula (II).

73. The method of claim 72, wherein the structure of said protein conjugate is represented by formula (VI): 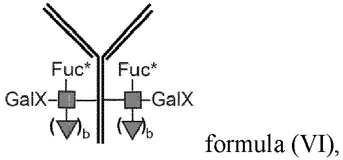 wherein ■ is the N-acetylglucosamine, ▼ is the fucose, the GalX is said optionally substituted galactose, 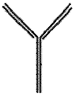 is a Fc domain-containing antibody and/or Fc fusion protein, b is 0 or 1, and said glycan chain is linked to the Fc domain of said Fc domain-containing antibody and/or Fc fusion protein.

74. The method of claim 73, further comprising the following steps:
treating the Fc domain-containing antibody and/or Fc fusion protein with an endoglycosidase to obtain said Fc domain-containing antibody and/or Fc fusion protein treated; and
contacting said treated antibody and/or Fc fusion protein with a UDP-GaIX in the presence of a suitable catalyst to obtain a protein having a glycan chain comprising a structure of formula (I), the structure of the protein being represented by formula (VII): 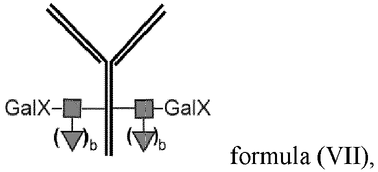
wherein ■ is the N-acetylglucosamine, ▼ is the fucose, the GalX is said optionally substituted galactose, 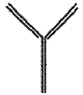 is the Fc domain-containing antibody and/or Fc fusion protein, b is 0 or 1, and said glycan chain is linked to the Fc domain of said Fc domain-containing antibody and/or Fc fusion protein.

75. The method of claim 73, further comprising the following steps:
treating the Fc domain-containing antibody and/or Fc fusion protein with an endoglycosidase and an α1,6 fucosidase to obtain said Fc domain-containing antibody and/or Fc fusion protein treated; and
contacting said treated antibody and/or Fc fusion protein with a UDP-GaIX in the presence of a suitable catalyst to obtain a protein having a glycan chain comprising a structure of formula (I), the structure of the protein being represented by formula (VII): 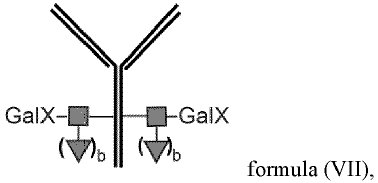
wherein ■ is the N-acetylglucosamine, ▼ is the fucose, the GalX is the optionally substituted galactose, 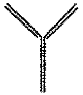 is the Fc domain-containing antibody and/or Fc fusion protein, b is 0, and said glycan chain is linked to the Fc domain of said Fc domain-containing antibody and/or Fc fusion protein.

76. The method of any one of claims 40-42, 50-61, and 63-75, further comprising the following step: reacting said obtained protein conjugate with a Y₁-L'-P₁' to obtain a protein conjugate comprising a biologically active substance and/or a pharmaceutically active substance P₁', wherein said Y₁ is a functional group capable of having a ligation reaction with said X₁, and L' is a linker.

77. A protein conjugate prepared by the method of any one of claims 20-76.

78. The protein conjugate of claim 77 having a MAR of about 3.2-4.0 or about 1.6-2.0.

79. A composition, comprising the protein conjugate of any one of claims 77-78.

80. The composition of claim 79 further comprising a pharmaceutically acceptable carrier.

81. A method for preventing, alleviating and/or treating a disease or a disorder, comprising: administering the protein conjugate of any one of claims 77-78 and/or the composition of any one of claims 79-80 to a subject in need.

82. A use of the protein conjugate of any one of claims 77-78 and/or the composition of any one of claims 79-80 in the preparation of a medicament, said medicament being used for preventing, alleviating and/or treating a disease or a disorder.
